# EUROPEAN PATENT APPLICATION

(11) **EP 3 299 018 A1**
(43) Date of publication of application: **28.03.2018**
(21) Application number: 17198877.7
(22) Date of filing: 04.04.2012
(51) Int. Cl.: A61K 31/496, A61K 31/00, A61K 31/135, A61K 31/138, A61K 31/15, A61K 31/165, A61K 31/343, A61K 31/381, A61K 31/425, A61K 31/5513, A61P 25/00, A61K 45/06, A61K 9/20

(54) **COMBINATIONS COMPRISING BREXPIPRAZOLE OR A SALT THEREOF AND A SECOND DRUG FOR USE IN THE TREATMENT OF AN CNS DISORDER**

(30) Priority: 05.04.2011 US 201161471911 P; 27.12.2011 US 201161580540 P
(62) Divisional of application: 12719460.3
(71) Applicant: Otsuka Pharmaceutical Co., Ltd., Tokyo 101-8535 (JP)
(72) Inventor: Hirose, Tsuyoshi, Osaka, 541-0045 (JP); Maeda, Kenji, Osaka, 541-0045 (JP); Kikuchi, Tetsuro, Osaka, 541-0045 (JP); Toda, Masafumi, Osaka, 541-0045 (JP)
(74) Representative: Hoffmann Eitle

(57) **Abstract**

The present invention provides a medicament having a wider treatment spectrum, causing a fewer side effects and superior in tolerability and safety, as compared to known typical antipsychotic agents and atypical antipsychotic agents. The present invention related to a medicament containing (I) a compound which is 7-[4-(4-benzo[b]thiophen-4-yl-piperazin-1-yl)butoxy]-1H-quinolin-2-one or a salt thereof, and (II) one or more antianxiety drugs, in combination.

## Description

### Technical Field

The present invention relates to a medicament comprising 7-[4-(4-benzo[b]thiophen-4-yl-piperazin-1-yl)butoxy]-1H-quinolin-2-one or a salt thereof, and at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, in combination.

### Background Art

It is known that 7-[4-(4-benzo[b]thiophen-4-yl-piperazin-1-yl)butoxy]-1H-quinolin-2-one (hereinafter to be referred to as Compound (I)) or a salt thereof has a dopamine D₂ receptor partial agonist activity (D₂ receptor partial agonist activity), a serotonin 5-HT_{2A} receptor antagonist activity (5-HT_{2A} receptor antagonist activity) and an adrenergic α₁ receptor antagonist activity (α₁ receptor antagonist activity) and, in addition thereto, concurrently has a serotonin uptake inhibitory action (or serotonin reuptake inhibitory action) (patent document 1 and patent document 2).

### Document List

### Patent Document

[patent document 1] WO2006/112464
[patent document 2] JP2008-115172

### Summary of the Invention

The present invention aims to provide a medicament having a broader treatment spectrum, causing a fewer side effects and superior in tolerability and safety, as compared to known typical antipsychotic agents and atypical antipsychotic agents.

The present inventors have conducted various studies in an attempt to solve the aforementioned problem and found that the above-mentioned problem can be solved by using at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, which are clinically used at present, and Compound (I) or a salt thereof in combination. The present invention has been completed based on such finding.

The present invention preferably provides medicaments shown in the following Item 1 to Item 20, a pharmaceutical composition shown in Item 21, a method for producing a pharmaceutical composition shown in Item 22, kits shown in Item 23 and Item 24, methods for preventing or treating a disease shown in Item 25 and Item 26, uses shown in Item 27 and Item 28, and medicaments shown in Item 29 and Item 30.

### Item 1.

A medicament comprising
(I) Compound (I) or a salt thereof and
(II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, in combination.

### Item 2.

The medicament of claim 1, which is a composition comprising
(I) Compound (I) or a salt thereof and
(II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug.

### Item 3.

The medicament of claim 1, which comprises
(I) a composition comprising Compound (I) or a salt thereof and
(II) a composition comprising at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, wherein the composition of (I) is used in combination with the composition of (II).

### Item 4.

The medicament of any one of Items 1 to 3, wherein said drug of (II) is a mood stabilizer.

### Item 5.

The medicament of Item 4, wherein said mood stabilizer is at least one drug selected from lithium, sodium valproate, divalproex sodium, carbamazepine, oxcarbazepine, zonisamide, lamotrigine, topiramate, gabapentin, levetiracetam, clonazepam, phenytoin, pregabalin, thyroid hormone, tiagabine, omega-3 fatty acid and salts thereof.

### Item 6.

The medicament of any one of Items 1 to 3, wherein said drug of (II) is a serotonin reuptake inhibitor.

### Item 7.

The medicament of Item 6, wherein said serotonin reuptake inhibitor is at least one drug selected from the group consisting of fluoxetine, citalopram, fluvoxamine, paroxetine, sertraline, escitalopram and salts thereof.

### Item 8.

The medicament of any one of Items 1 to 3, wherein said drug of (II) is a norepinephrine reuptake inhibitor.

### Item 9.

The medicament of Item 8, wherein said norepinephrine reuptake inhibitor is at least one drug selected from the group consisting of reboxetine, atomoxetine, bupropion and salts thereof.

### Item 10.

The medicament of any one of Items 1 to 3, wherein said drug of (II) is a serotonin and norepinephrine reuptake inhibitor.

### Item 11.

The medicament of Item 10, wherein said serotonin and norepinephrine reuptake inhibitor is at least one drug selected from the group consisting of venlafaxine, duloxetine, milnacipran, desvenlafaxine and salts thereof.

### Item 12.

The medicament of any one of Items 1 to 3, wherein said drug of (II) is a noradrenergic and specific serotonergic antidepressant.

### Item 13.

The medicament of Item 12, wherein said noradrenergic and specific serotonergic antidepressant is at least one drug selected from the group consisting of mirtazapine and a salt thereof.

### Item 14.

The medicament of any one of Items 1 to 3, wherein said drug of (II) is an antianxiety drug.

### Item 15.

The medicament of Item 14, wherein said antianxiety drug is at least one drug selected from the group consisting of a benzodiazepine antianxiety drug and a serotonin 5-HT1A receptor agonist antianxiety drug.

### Item 16.

The medicament of Item 15, wherein said benzodiazepine antianxiety drug is at least one drug selected from the group consisting of diazepam, lorazepam, chlordiazepoxide, cloxazolam, clotiazepam, alprazolam, etizolam, oxazolam and salts thereof, and said serotonin 5-HT1A receptor agonist antianxiety drug is at least one drug selected from the group consisting of tandospirone, buspirone and salts thereof.

### Item 17.

The medicament of any one of Items 1 to 3, wherein said drug of (II) is an anti-ADHD drug.

### Item 18.

The medicament of Item 17, wherein said anti-ADHD drug is at least one drug selected from the group consisting of methylphenidate, dexmethylphenidate, atomoxetine, dextroamphetamine, mixed amphetamine salts, modafinil, guanfacine, clonidine and salts thereof.

### Item 19.

The medicament of any one of Items 1 to 18 for use in the prophylaxis or treatment of a central nervous system disease.

### Item 20.

The medicament of Item 19, wherein the central nervous system disease is a central nervous system disease selected from the group consisting of schizophrenia, treatment-resistant, refractory or chronic schizophrenia, emotional disturbance, psychotic disorder, mood disorder, bipolar disorder (e.g., bipolar disorder type I and bipolar disorder type II), mania, depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, acute stress disorder), somatoform disorder (e.g., hysteria, somatization disorder, conversion disorder, pain disorder, hypochondriasis), factitious disorder, dissociative disorder, sexual disorder (e.g., sexual dysfunction, sexual desire disorder, sexual arousal disorder, erectile dysfunction), eating disorder (e.g., anorexia nervosa, bulimia nervosa), sleep disorder, adjustment disorder, substance-related disorder (e.g., alcohol abuse, alcohol intoxication and drug addiction, stimulant intoxication, narcotism), anhedonia (e.g., iatrogenic anhedonia, anhedonia of a psychic or mental cause, anhedonia associated with depression, anhedonia associated with schizophrenia), delirium, cognitive impairment, cognitive impairment associated with Alzheimer's disease, Parkinson's disease, and other neurodegenerative diseases, cognitive impairment caused by Alzheimer's disease, Parkinson's disease and associated neurodegenerative diseases, cognitive impairment in schizophrenia, cognitive impairment caused by treatment-resistant, refractory or chronic schizophrenia, vomiting, motion sickness, obesity, migraine, pain, mental retardation, autistic disorder (autism), Tourette's disorder, tic disorder, attention deficit hyperactivity disorder, conduct disorder and Down's syndrome.

### Item 21.

A pharmaceutical composition comprising the medicament of any one of Items 1 to 18 and at least one pharmacologically acceptable carrier.

### Item 22.

A method for producing a pharmaceutical composition, comprising mixing the medicament of any one of Items 1 to 18 with a pharmacologically acceptable carrier.

### Item 23.

A kit comprising
(I) a medicament containing Compound (I) or a salt thereof and
(II) a medicament containing at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug.

### Item 24.

The kit of Item 23, which is a kit for use in the prophylaxis or treatment of a central nervous system disease.

### Item 25.

A method for preventing or treating a disease in a mammal, comprising
administering an effective amount of Compound (I) or a salt thereof to the mammal, and
administering an effective amount of (II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, to the mammal.

### Item 26.

The method of Item 25, wherein said disease is a central nervous system disease.

### Item 27.

Use of Compound (I) or a salt thereof and (II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, for the production of a medicament for the treatment of a central nervous system disease by a combination treatment using said drug of (II) together with Compound (I) or a salt thereof, wherein Compound (I) or a salt thereof and said drug of (II) are formulated as a part of a single drug, or formulated as individual drugs to be administered simultaneously or at different time points.

### Item 28.

Use of (II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, together with Compound (I) or a salt thereof, for the treatment of a central nervous system disease.

### Item 29.

A medicament for use in the prophylaxis or treatment of a central nervous system disease comprising Compound (I) or a salt thereof and at least one drug selected from the group consisting of lithium, sodium valproate, divalproex sodium, carbamazepine, oxcarbazepine, zonisamide, lamotrigine, topiramate, gabapentin, levetiracetam, clonazepam, phenytoin, pregabalin, thyroid hormone, tiagabine, omega-3 fatty acid, fluoxetine, citalopram, fluvoxamine, paroxetine, sertraline, escitalopram, reboxetine, atomoxetine, bupropion, venlafaxine, duloxetine, milnacipran, desvenlafaxine, mirtazapine, diazepam, lorazepam, chlordiazepoxide, cloxazolam, clotiazepam, alprazolam, etizolam, oxazolam, tandospirone, buspirone, methylphenidate, dexmethylphenidate, dextroamphetamine, mixed amphetamine salts, modafinil, guanfacine, clonidine and salts thereof, in combination.

### Item 30.

The medicament of Item 29, wherein the central nervous system disease is a central nervous system disease selected from the group consisting of schizophrenia, treatment-resistant, refractory or chronic schizophrenia, emotional disturbance, psychotic disorder, mood disorder, bipolar disorder (e.g., bipolar disorder type I and bipolar disorder type II), mania, depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, acute stress disorder), somatoform disorder (e.g., hysteria, somatization disorder, conversion disorder, pain disorder, hypochondriasis), factitious disorder, dissociative disorder, sexual disorder (e.g., sexual dysfunction, sexual desire disorder, sexual arousal disorder, erectile dysfunction), eating disorder (e.g., anorexia nervosa, bulimia nervosa), sleep disorder, adjustment disorder, substance-related disorder (e.g., alcohol abuse, alcohol intoxication and drug addiction, stimulant intoxication, narcotism), anhedonia (e.g., iatrogenic anhedonia, anhedonia of a psychic or mental cause, anhedonia associated with depression, anhedonia associated with schizophrenia), delirium, cognitive impairment, cognitive impairment associated with Alzheimer's disease, Parkinson's disease, and other neurodegenerative diseases, cognitive impairment caused by Alzheimer's disease, Parkinson's disease and associated neurodegenerative diseases, cognitive impairment in schizophrenia, cognitive impairment caused by treatment-resistant, refractory or chronic schizophrenia, vomiting, motion sickness, obesity, migraine, pain, mental retardation, autistic disorder (autism), Tourette's disorder, tic disorder, attention deficit hyperactivity disorder, conduct disorder and Down's syndrome. Description of Embodiments

Examples of preferable salts of 7-[4-(4-benzo[b]thiophen-4-yl-piperazin-1-yl)butoxy]-1H-quinolin-2-one usable in the present invention include salts of inorganic acids such as sulfate, nitrate, hydrochloride, phosphate, hydrobromide and the like, salts of organic acids such as acetate, sulfonates such as p-toluenesulfonate, methanesulfonate, ethanesulfonate and the like, oxalate, maleate, fumarate, maleate, tartrate, citrate, succinate, benzoate and the like.

Compound (I) or a salt thereof usable in the present invention is also encompasses the same isotopically-labeled compounds, wherein one or plural atoms is(are) replaced by one or plural atoms having a particular atomic mass or mass number. Examples of the isotope that can be incorporated into Compound (I) or a salt thereof include hydrogen, carbon, nitrogen, oxygen, sulfur, fluorine and chlorine isotopes such as ²H, ³H, ¹³C, ¹⁴C, ¹⁵N, ¹⁸O, ¹⁷O, ¹⁸F, ³⁶Cl and the like. Certain isotopically-labeled Compound (I) or a salt thereof, which contains the above-mentioned isotope and/or other isotope of other atom, for example, Compound (I) or a salt thereof incorporating a radioactive isotope such as ³H, ¹⁴C and the like, is useful for drug tissue distribution assay and/or substrate tissue distribution assay. Tritiated (i.e., ³H) or carbon-14 (i.e., ¹⁴C) isotope are particularly preferred because of easiness of preparation and detectability. Furthermore, substitution with a heavier isotope such as deuterium (i.e., ²H) and the like is expected to provide improved metabolic stability and particular therapeutic advantage attributable to increased in vivo half-life or decreased amount of necessary administration. An isotopically-labeled compound of Compound (I) or a salt thereof can be generally prepared according to the method disclosed in WO2006/112464, by substituting a non-isotopically-labeled reagent with an easily available isotopically-labeled reagent.

Compound (I) or a salt thereof, a production method thereof, a dose to be used thereof and the like are disclosed in WO2006/112464, and the disclosure thereof constitutes a part of the present specification by reference.

Examples of the aforementioned drug of (II) that can be used in combination with Compound (I) or a salt thereof in the present invention (hereinafter to be referred to as drug (II)) include a drug selected from the following. Said drug (II) may be used alone or two or more kinds thereof may be used in combination. A combined use of Compound (I) or a salt thereof and one drug selected from the following is preferable.

### (1) mood stabilizer

As a mood stabilizer, compounds that function as a mood stabilizer can be widely used, and they are known to those of ordinary skill in the art.

As a non-limitative list of the mood stabilizers usable in the present invention, lithium, sodium valproate, divalproex sodium, carbamazepine, oxcarbazepine, zonisamide, lamotrigine, topiramate, gabapentin, levetiracetam, clonazepam, phenytoin, pregabalin, thyroid hormone, tiagabine, omega-3 fatty acid and salts thereof can be mentioned. Preferably, lamotrigine, zonisamide, topiramate, lithium, sodium valproate, carbamazepine and salts thereof can be mentioned.

### (2) serotonin reuptake inhibitor

As a serotonin reuptake inhibitor, known compounds can be widely used as long as they function as a serotonin reuptake inhibitor.

Among the serotonin reuptake inhibitors, those showing an IC50 value (drug concentration necessary for inhibiting serotonin reuptake by 50%) of about 1000 nM or less, by the method of Wong et al. (Neuropsychopharmacology, 8, pp 337-344 (1993)), which is a conventional standard pharmacological assay, are preferable.

Examples of such serotonin reuptake inhibitor include fluvoxamine, fluoxetine, paroxetine, sertraline, citalopram, escitalopram, trazodone, nefazodone and salts thereof, and the like. Preferable examples thereof include fluvoxamine, fluoxetine, paroxetine, sertraline, escitalopram and salts thereof.

### (3) norepinephrine reuptake inhibitor

As a norepinephrine reuptake inhibitor, known compounds can be widely used as long as they function as a norepinephrine reuptake inhibitor. Examples of such norepinephrine reuptake inhibitor include reboxetine, atomoxetine, bupropion and salts thereof. Preferred are reboxetine, atomoxetine and salts thereof.

### (4) serotonin and norepinephrine reuptake inhibitor

As a serotonin and norepinephrine reuptake inhibitor, known compounds can be widely used as long as they function as a serotonin and norepinephrine reuptake inhibitor. Examples of such serotonin and norepinephrine reuptake inhibitor include venlafaxine, duloxetine, milnacipran, desvenlafaxine and salts thereof, and the like.

### (5) noradrenergic and specific serotonergic antidepressant

Examples of the noradrenergic and specific serotonergic antidepressant include mirtazapine and a salt thereof, and the like.

### (6) antianxiety drug

As a non-limitative list of the antianxiety drug usable in the present invention, benzodiazepine antianxiety drugs such as diazepam, lorazepam, chlordiazepoxide, cloxazolam, clotiazepam, alprazolam, etizolam, oxazolam and salts thereof, and the like, serotonin 5-HT1A receptor agonist antianxiety drugs including tandospirone, buspirone and salts thereof, and the like can be mentioned.

### (7) tricyclic antidepressant

As a non-limitative list of the tricyclic antidepressant usable in the present invention, amitriptyline, imipramine, doxepin, clomipramine, nortriptyline, amoxapine, trimipramine, lofepramine, dosulepin, protriptyline, desipramine and salts thereof, and the like can be mentioned.

### (8) tetracyclic antidepressant

As a non-limitative list of the tetracyclic antidepressant usable in the present invention, maprotiline, mianserin, setiptiline, mirtazapine and salts thereof, and the like can be mentioned.

### (9) antipsychotic drug

As a non-limitative list of the antipsychotic drug usable in the present invention, aripiprazole, olanzapine, quetiapine, risperidone, ziprasidone, amisulpride, clozapine, chlorpromazine, haloperidol decanoate, paliperidone, mosapramine, zotepine, blonanserin, asenapine, iloperidone, cariprazine and salts thereof, and the like can be mentioned.

### (10) anti-ADHD drug

As a non-limitative list of an anti-ADHD drug usable in the present invention, methylphenidate, dexmethylphenidate, atomoxetine, dextroamphetamine, mixed amphetamine salts, modafinil, guanfacine, clonidine and salts thereof, and the like can be mentioned.

A drug selected from the group consisting of the above-mentioned (1) mood stabilizer, (2) serotonin reuptake inhibitor, (3) norepinephrine reuptake inhibitor, (4) serotonin and norepinephrine reuptake inhibitor, (5) noradrenergic and specific serotonergic antidepressant, (6) antianxiety drug (7) tricyclic antidepressant, (8) tetracyclic antidepressant, (9) antipsychotic drug and (10) anti-ADHD drug may take any form of free base or salt (acid addition salt etc.). In addition, these drugs may be racemates, or R or S enantiomers. These drugs may be used as a single drug, or two or more kinds thereof may be used in combination as necessary. Use of a single drug is preferable.

These drugs can easily form an acid addition salt with a pharmaceutically acceptable acid. Examples of such acid include inorganic acids such as sulfuric acid, nitric acid, hydrochloric acid, phosphoric acid, hydrobromic acid and the like; and organic acids such as acetic acid, p-toluenesulfonic acid, methanesulfonic acid, oxalic acid, maleic acid, fumaric acid, malic acid, tartaric acid, citric acid, benzoic acid and the like. These acid addition salts can also be used as an active ingredient compound in the present invention, in the same manner as a drug in a free form.

Among these drugs, a compound having an acidic group can easily form a salt when reacted with a pharmaceutically acceptable basic compound. Examples of such basic compound include metal hydroxides such as sodium hydroxide, potassium hydroxide, lithium hydroxide, calcium hydroxide and the like; alkali metal carbonates or bicarbonates such as sodium carbonate, potassium carbonate, sodium hydrogen carbonate, potassium hydrogen carbonate and the like; metal alcoholates such as sodium methylate, potassium ethylate and the like; and the like.

The thus-obtained drug in a salt form can be separated from the reaction system according to a general separation method, and further purified. Examples of the separation and purification means include distillation, solvent extraction, dilution, recrystallization, column chromatography, ion exchange chromatography, gel chromatography, affinity chromatography, preparative thin layer chromatography and the like.

### Preferable combination of Compound (I) or a salt thereof and drug (II) usable in combination

When Compound (I) or a salt thereof is combined with at least one mood stabilizer, the following combinations are preferable: combination of Compound (I) or a salt thereof and carbamazepine; combination of Compound (I) or a salt thereof and oxcarbazepine; combination of Compound (I) or a salt thereof and zonisamide; combination of Compound (I) or a salt thereof and lamotrigine; combination of Compound (I) or a salt thereof and topiramate; combination of Compound (I) or a salt thereof and gabapentin; combination of Compound (I) or a salt thereof and levetiracetam; combination of Compound (I) or a salt thereof and clonazepam; combination of Compound (I) or a salt thereof and lithium; combination of Compound (I) or a salt thereof and sodium valproate; and combination of Compound (I) or a salt thereof and pregabalin.

When Compound (I) or a salt thereof is combined with at least one serotonin reuptake inhibitor, the following combinations are preferable: combination of Compound (I) or a salt thereof and citalopram; combination of Compound (I) or a salt thereof and fluvoxamine; combination of Compound (I) or a salt thereof and fluoxetine; combination of Compound (I) or a salt thereof and paroxetine; combination of Compound (I) or a salt thereof and sertraline; and combination of Compound (I) or a salt thereof and escitalopram.

When Compound (I) or a salt thereof is combined with at least one norepinephrine reuptake inhibitor, the following combinations are preferable: combination of Compound (I) or a salt thereof and reboxetine; and combination of Compound (I) or a salt thereof and atomoxetine.

When Compound (I) or a salt thereof is combined with at least one serotonin and norepinephrine reuptake inhibitor, the following combinations are preferable: combination of Compound (I) or a salt thereof and venlafaxine; combination of Compound (I) or a salt thereof and duloxetine; combination of Compound (I) or a salt thereof and desvenlafaxine; and combination of Compound (I) or a salt thereof and milnacipran.

When Compound (I) or a salt thereof is combined with at least one noradrenergic and specific serotonergic antidepressant, the following combination is preferable: combination of Compound (I) or a salt thereof and mirtazapine.

When Compound (I) or a salt thereof is combined with at least one antianxiety drug, the following combinations are preferable: combination of Compound (I) or a salt thereof and benzodiazepine antianxiety drug (diazepam, lorazepam, chlordiazepoxide, cloxazolam, clotiazepam, alprazolam, etizolam, oxazolam etc.); and combination of Compound (I) or a salt thereof and serotonin 5-HT1A receptor agonist antianxiety drug (tandospirone, buspirone etc.).

A pharmaceutical composition containing the above-mentioned preferable combination provides a superior effect. Therefore, such composition causes a fewer side effects and has a superior safety profile.

Compound (I) or a salt thereof and drug (II) may be administered orally or parenterally.

In the present specification, when a medicament comprising Compound (I) or a salt thereof and drug (II) in combination is used, the administration period of Compound (I) or a salt thereof and drug (II) is not limited, and Compound (I) or a salt thereof and drug (II) may be simultaneously formulated into a single preparation, or Compound (I) or a salt thereof or a pharmaceutical composition thereof and drug (II) or a pharmaceutical composition thereof may be administered to a subject of administration simultaneously or in a staggered manner. When Compound (I) or a salt thereof and drug (II) are administered, they may be administered simultaneously. Alternatively, drug (II) may be administered in advance, and then Compound (I) or a salt thereof may be administered, or Compound (I) or a salt thereof may be administered in advance, and then drug (II) may be administered. For administration in a staggered manner, the time difference varies depending on the active ingredient to be administered, dosage form and administration method. For example, when drug (II) is to be administered in advance, a method including administering Compound (I) or a salt thereof within 1 min - 3 days, preferably 10 min - 1 day, more preferably 15 min - 1 hr, after the administration of drug (II) can be mentioned. The dose of drug (II) may be similar to the dose clinically used, and can be appropriately determined according to the subject of administration, administration route, disease, combination and the like. The administration form of the medicament of the present invention is not particularly limited, and Compound (I) or a salt thereof and drug (II) only need to be combined on administration. Examples of such administration form include (1) administration of a single preparation obtained by simultaneously formulating Compound (I) or a salt thereof and drug (II), (2) simultaneous administration of two kinds of preparations obtained by separately formulating Compound (I) or a salt thereof and drug (II) by the same administration route, (3) administration of two kinds of preparations obtained by separately formulating Compound (I) or a salt thereof and drug (II) by the same administration route in a staggered manner (e.g., administration in the order of Compound (I) or a salt thereof; one or more kinds of drug (II), or administration in the reverse order), (4) simultaneous administration of two kinds of preparations obtained by separately formulating Compound (I) or a salt thereof and drug (II) by different administration routes, (5) administration of one or more kinds of preparations obtained by separately formulating Compound (I) or a salt thereof and drug (II) by different administration routes in a staggered manner (e.g., administration in the order of Compound (I) or a salt thereof; one or more kinds of drug (II), or in the reverse order) and the like.

The medicaments of the present invention comprising Compound (I) or a salt thereof, drug (II) and/or Compound (I) or a salt thereof and drug (II) in combination, which are constituent components of the present invention, show low toxicity and, for example, Compound (I) or a salt thereof and/or drug (II) can be mixed with a pharmacologically acceptable carrier according to a known method to give a pharmaceutical composition, such as tablets (including sugar-coated tablet, film-coated tablet), powders, granules, capsules (including soft capsule), liquids, injections, suppositories, sustained-release preparations and the like, which can be safely administered orally or parenterally (e.g., local, rectum, vein, and the like). An injection can be administered by intravenous, intramuscular, subcutaneous or intraorgan administration or directly to the lesion. As a pharmacologically acceptable carrier which may be used for producing the pharmaceutical composition of the present invention, excipient, disintegrant, binder, fluidizer, lubricant, coating agent, colorant, suspending agent, sweetening agent or surfactant is appropriately used, and a general pharmaceutical preparation is formed according to a known method. Examples of the form of the pharmaceutical preparation include powder, tablet, pill, capsule and the like.

Examples of the excipient include lactose, anhydrous lactose, purified sucrose, sucrose, D-mannitol, D-sorbitol, xylitol, erythritol, dextrin, crystalline cellulose, microcrystalline cellulose, cornstarch, potato starch, anhydrous calcium hydrogen phosphate and the like.

Examples of the disintegrant include sodium carboxymethyl starch, carmellose, carmellose calcium, carmellose sodium, croscarmellose sodium, crospovidone, low-substituted hydroxypropylcellulose, partially pregelatinized starch and the like.

Examples of the binder include hydroxypropylcellulose, hydroxypropylmethylcellulose, polyvinylpyrrolidone, pregelatinized starch, syrup, starch syrup and the like.

Examples of the fluidizer include light anhydrous silicic acid, synthetic aluminum silicate, hydrated silicon dioxide, calcium stearate, magnesium aluminometasilicate, talc and the like.

Examples of the lubricant include magnesium stearate, calcium stearate, magnesium silicate, magnesium oxide, talc, hydrogenated oil, sucrose ester of fatty acid, sodium stearyl fumarate and the like.

Examples of the coating agent include hydroxypropylmethylcellulose, polyvinyl alcohol, polysorbate, macrogol, talc and the like.

Examples of the colorant include yellow iron sesquioxide, brown iron oxide, iron sesquioxide, black iron oxide, titanium oxide, Food Blue No. 1, Food Red No. 2, Food Red No. 3, Food Yellow No. 4 and the like.

Examples of the suspending agent include polysorbate, polyethylene glycol, gum arabic, glycerol, gelatin and the like.

Examples of the sweetening agent include aspartame, saccharin, saccharin sodium, starch syrup, fructose and the like.

Examples of the surfactant include sodium lauryl sulfate, polysorbate, polyoxyethylene hydrogenated castor oil and the like.

A capsule is prepared by filling a hard capsule such as gelatin capsule, hydroxypropylmethylcellulose capsule, polyvinyl alcohol capsule and the like or a soft capsule based on gelatin, according to a known method. Conventional various organic or inorganic carrier substances can be used as preparation starting materials and examples thereof include excipient, lubricant, binder and disintegrant for solid preparations, and solvent, solubilizing agent, suspending agent, isotonicity agent, buffering agent and soothing agent for liquid preparations and the like. Furthermore, where necessary, additives such as general preservative, antioxidant, colorant, sweetening agent, adsorbent, wetting agent and the like can be appropriately used in an appropriate amount.

### Dose

The dose of drug (II) to be used in the present invention is determined in consideration of the properties of each constituent drug to be used in combination, properties of the drug after combination, and the condition of the patients. As shown above, Compound (I) or a salt thereof and drug (II) may be separately administered without being combined in one composition. As the general outline of the dose, for example, the following guideline can be applied.

In the following description of the dose, for example, "about 0.05 - about 50 mg/2 times/1 day" means administration of about 0.05 - about 50 mg per administration twice a day.

Compound (I) or a salt thereof: generally about 0.1 - about 100 mg/1 time/1 day (or, about 0.05 - about 50 mg/2 times/1 day), preferably about 1 - about 3 mg/1 time/1 day (or, about 0.5 - about 1.5 mg/2 times/1 day).

Compound (I) or a salt thereof can be combined with at least one (drug (II)) selected from the following mood stabilizer, serotonin reuptake inhibitor, norepinephrine reuptake inhibitor, serotonin and norepinephrine reuptake inhibitor, noradrenergic and specific serotonergic antidepressant, antianxiety drug, tricyclic antidepressant, tetracyclic antidepressant, antipsychotic drug and anti-ADHD drug, within the designated dose range, or can be separately administered:
(1) mood stabilizer
   lithium: generally about 200 - about 2400 mg/1 day, or about 200 - about 1200 mg/2 - 3 times/1 day.
   sodium valproate: generally about 400 - about 2000 mg/1 day.
   divalproex sodium: generally about 250 - about 3600 mg/1 day.
   carbamazepine: generally about 100 - about 1600 mg/1 day.
   oxcarbazepine: generally about 600 - about 2400 mg/1 day.
   zonisamide: generally about 100 - about 600 mg/1 day.
   lamotrigine: generally about 25 - about 500 mg/1 day,
   preferably about 100 - about 400 mg/1 day.
   topiramate: generally about 50 - about 600 mg/1 day.
   gabapentin: generally about 600 - about 2400 mg/1 time/1 day.
   levetiracetam: generally about 250 - about 3000 mg/1 day.
   clonazepam: generally about 0.1 - about 6 mg/1 day.
   phenytoin: generally about 20 - about 300 mg/1 day.
   pregabalin: generally about 10 - about 1000 mg/1 - 3 times/1 day, preferably about 50 - about 600 mg/2 - 3 times/1 day. tiagabine: generally about 4 - about 56 mg/1 day.
(2) serotonin reuptake inhibitor
   fluoxetine: generally about 1 - about 80 mg/1 time/1 day, preferably about 10 - about 40 mg/1 time/1 day.
   citalopram: generally about 5 - about 50 mg/1 time/1 day, preferably about 10 - about 30 mg/1 time/1 day.
   fluvoxamine: generally about 20 - about 500 mg/1 time/1 day, preferably about 50 - about 300 mg/1 - 2 times/1 day.
   paroxetine: generally about 10 - about 100 mg/1 time/1 day,
   preferably about 20 - about 50 mg/1 time/1 day.
   sertraline: generally about 20 - about 500 mg/1 time/1 day,
   preferably about 50 - about 200 mg/1 time/1 day.
   escitalopram: generally about 5 - about 30 mg/1 time/1 day, preferably about 10 - about 20 mg/1 time/1 day.
(3) norepinephrine reuptake inhibitor
   reboxetine: generally about 1 - about 30 mg/1 - 4 times/1 day, preferably about 5 - about 30 mg/2 times/1 day.
   atomoxetine: generally about 5 - about 120 mg/1 - 2 times/1 day, preferably about 0.5 - about 1.2 mg/kg/1 time/1 day (or about 0.25 - about 0.6 mg/kg/2 times/1 day).
   bupropion: generally about 10 - about 600 mg/1 - 3 times/1 day, preferably about 150 - about 450 mg/1 time/1 day (or about 75 - about 225 mg/2 times/1 day).
(4) serotonin and norepinephrine reuptake inhibitor
   venlafaxine: generally about 10 - about 300 mg/1 - 3 times/1 day, preferably about 37.5 - about 225 mg/1 time/1 day (or about 37.5 - about 75 mg/2 - 3 times/1 day).
   desvenlafaxine: generally about 10 - about 150 mg/1 time/1 day, preferably about 50 - about 100 mg/1 time/1 day.
   duloxetine: generally about 1 - about 100 mg/1 time/1 day, preferably about 20 - about 60 mg/1 time/1 day.
   milnacipran: generally about 10 - about 100 mg/1 - 2 times/1 day, preferably about 25 - about 50 mg/2 times/1 day.
(5) noradrenergic and specific serotonergic antidepressant
   mirtazapine: generally about 5 - about 100 mg/1 time/1 day, preferably about 15 - about 45 mg/1 time/1 day.
(6) antianxiety drug
   diazepam: generally about 1 - about 15 mg/1 - 4 times/1 day, preferably about 2 - about 5 mg/2 - 4 times/1 day (in principle within 15 mg/1 day).
   lorazepam: generally about 0.1 - about 30 mg/1 - 3 times/1 day, preferably about 0.33 - about 1 mg/2 - 3 times/1 day.
   chlordiazepoxide: generally about 2.5 - about 100 mg/1 - 3 times/1 day, preferably about 2.5 - about 20 mg/2 - 4 times/1 day.
   cloxazolam: generally about 0.1 - about 30 mg/1 - 3 times/1 day, preferably about 1 - about 4 mg/3 times/1 day.
   clotiazepam: generally about 1 - about 100 mg/1 - 3 times/1 day, preferably about 5 - about 10 mg/3 times/1 day.
   alprazolam: generally about 0.1 - about 10 mg/1 - 3 times/1 day, preferably about 0.4 - about 0.8 mg/3 times/1 day.
   etizolam: generally about 0.1 - about 10 mg/1 - 3 times/1 day, preferably about 1 mg/3 times/1 day.
   oxazolam: generally about 1 - about 60 mg/1 - 3 times/1 day, preferably about 3.3 - about 6.7 mg/3 times/1 day.
   tandospirone: generally about 3 - about 120 mg/1 - 3 times/1 day, preferably about 10 - about 20 mg/3 times/1 day.
   buspirone: generally about 1.5 - about 100 mg/1 - 2 times/1 day, preferably about 7.5 - about 30 mg/2 times/1 day.
(7) tricyclic antidepressant
   amitriptyline: The initial dose is 30-75 mg/day, gradually increased to 150 mg/day, orally in divided doses. In rare cases, the dose can be increased to 300 mg.
   imipramine: The initial dose is 25-75 mg/day, gradually increased to 200 mg/day, orally in divided doses. In rare cases, the dose can be increased to 300 mg.
   doxepin: The initial dose is 25-50 mg, orally in 2 to 3 divided doses per day. In rare cases, the dose can be gradually increased to 300 mg/day.
   clomipramine: 50-100 mg/day in 1 to 3 divided doses. Maximum daily dose is up to 220 mg.
   nortriptyline: 10-25 mg/day, orally in 3 divided doses, or 10-25 mg/day, orally in 2 divided doses. Maximum dose is up to 150 mg/day, orally in 2 to 3 divided doses.
   amoxapine: The initial dose is 25-75 mg/day, gradually increased to 200 mg/day, orally in divided doses. In rare cases, the dose can be increased to 300 mg.
   trimipramine: The initial dose is 50-100 mg/day, gradually increased to 200 mg/day, orally in divided doses. In rare cases, the dose can be increased to 300 mg.
   lofepramine: 10-25 mg, orally in 2 to 3 divided doses per day. The dose can be gradually increased to 150 mg/day.
   dosulepin: 75-150 mg, orally in 2 to 3 divided doses per day.
   protriptyline: 15-40 mg, orally in 3 to 4 divided doses per day. In rare cases, the dose can be gradually increased to 60 mg/day.
   desipramine: The initial dose is 50-75 mg/day, gradually increased to 150 mg/day, orally in divided doses. In rare cases, the dose can be gradually increased to 200 mg/day.
(8) tetracyclic antidepressant
   maprotiline: 30-75 mg, orally in 2 to 3 divided doses per day. mianserin: The initial dose is 30 mg/day, gradually increased to 60 mg/day, orally in divided doses.
   setiptiline: The initial dose is 3 mg/day, gradually increased to 6 mg/day, orally in divided doses.
   mirtazapine: generally about 5 - about 100 mg/1 time/1 day, preferably about 15 - about 45 mg/1 time/1 day.
(9) antipsychotic drug
   aripiprazole: The initial dose is 6-12 mg/day, and the maintenance dose is 6-24 mg/day, orally in single or 2 divided doses. The daily dose should not exceed 30 mg.
   olanzapine: for schizophrenia, the initial dose is 5-10 mg/day, and the maintenance dose is 10 mg/day, orally administered once a day. The daily dose should not exceed 20 mg.
   quetiapine: The initial dose is 25-75 mg/day, orally in 2 to 3 divided doses. Generally, daily dose is 150-600 mg. The daily dose should not exceed 750 mg.
   risperidone: The initial dose is 1 mg, orally in 2 divided doses per day, and the maintenance dose is 2-6 mg, orally in 2 divided doses per day. The daily dose should not exceed 12 mg. ziprasidone: 40-60 mg/day, orally in 2 divided doses. The daily dose should not exceed 80 mg.
   amisulpride: 50-800 mg/day, orally in divided doses.
   clozapine: The initial dose is 25 mg on day 1, and 50 mg on day 2, orally administered once a day. On day 3 and thereafter, the dose can be increased by 25 mg per day depending on symptoms, up to 200 mg over 3 weeks in principle. If the daily dose exceeds 50 mg, it should be administered orally in 2 to 3 divided doses. The maintenance dose is 200-400 mg/day, orally in 2 to 3 divided doses. The interval between each dose increment should be at least 4 days, and the dose increase should not exceed 100 mg/day. Maximum daily dose is up to 600 mg.
   chlorpromazine: 10-450 mg/day, orally in divided doses.
   haloperidol decanoate: a single dose of 50-150 mg, intramuscularly at 4-week intervals. A standard initial dose is 10-15 times the daily dose of oral haloperidol, and it should not exceed 100 mg.
   paliperidone: 6 mg, orally administered once a day. The dose can be properly increased or decreased within a range not exceeding 12 mg/day. However, the interval between each dose increment should be at least 5 days, and the daily dose should be increased by 3 mg.
   mosapramine: 30-150 mg/day, orally in 3 divided doses. The dose can be increased to 300 mg/day.
   zotepine: 75-150 mg/day, orally in divided doses. The dose can be increased to 450 mg/day.
   blonanserin: The initial dose is 4 mg per administration, orally twice a day, gradually increased. The maintenance dose is 8-16 mg/day, orally in 2 divided doses. Maximum dose should not exceed 24 mg/day.
   asenapine: 5-10 mg, sublingually in 2 divided doses per day.
   iloperidone: The initial dose is 1 mg/day, orally in 2 divided doses. On day 2, 2 mg/day, orally in 2 divided doses.
      Thereafter, the dose can be gradually increased by 2 mg per day up to 12 mg.
(10) anti-ADHD drug
   methylphenidate: generally about 10 - about 100 mg/1 - 2 times/1 day, preferably about 10 - about 72 mg/1 time/1 day. dexmethylphenidate: generally about 1 - about 50 mg/1 time/1 day, preferably about 5 - about 40 mg/1 time/1 day. atomoxetine: generally about 5 - about 120 mg/1 - 2 times/1 day, preferably about 0.5 - about 1.2 mg/kg/1 time/1 day (or about 0.25 - about 0.6 mg/kg/2 times/1 day).
   dextroamphetamine: generally about 1 - about 100 mg/1 time/1 day, preferably about 5 - about 60 mg/1 time/1 day (or about 2.5 - about 30 mg/2 times/1 day).
   mixed amphetamine salts: generally about 1 - about 50 mg/1 time/1 day, preferably about 2.5 - about 40 mg/1 time/1 day.
   modafinil: generally about 10 - about 500 mg/1 - 3 times/1 day, preferably about 200 - about 400 mg/1 time/1 day.
   guanfacine: generally about 0.1 - about 10 mg/1 time/1 day, preferably about 1 - about 4 mg/1 time/1 day.
   clonidine: generally about 0.05 - about 1 mg/1 - 2 times/1 day, preferably about 0.1 - about 0.4 mg/1 - 2 times/1 day.

In the present invention, the proportion of Compound (I) or a salt thereof and drug (II) to be used may be generally about 0.01 - about 500 parts by weight, preferably about 0.1 - about 100 parts by weight, of the latter relative to 1 part by weight of the former.

The mixing ratio of Compound (I) or a salt thereof and drug (II) in the therapeutic drug of the present invention can be appropriately determined according to the subject of administration, administration route, disease and the like. For example, while the total proportion of Compound (I) or a salt thereof and drug (II) in the therapeutic drug of the present invention varies depending on the preparation form, it is generally about 0.01 - about 99.99 wt%, preferably about 0.1 - about 99.9 wt%, more preferably about 1 - about 30 wt%, relative to the whole preparation. The above-mentioned pharmacologically acceptable carrier is used for the remaining part.

In addition, when Compound (I) or a salt thereof and drug (II) are to be separately formulated, a similar content may be used.

The present invention may also be in the form of a kit comprising a medicament containing Compound (I) or a salt thereof and a medicament containing drug (II), which are separately formulated. The kind of the preparation is not particularly limited, and tablets (including sugar-coated tablet, film-coated tablet), powder, granule, capsule (including soft capsule), liquid, injection, suppository, sustained-release preparation and the like can be mentioned. Preferred is, for example, a kit comprising an oral preparation containing Compound (I) or a salt thereof (tablet, powder, granule, capsule or liquid), and an oral preparation containing drug (II) (tablet, powder, granule, capsule or liquid).

While the dose of the medicament or pharmaceutical composition of the present invention varies depending on a kind of Compound (I) or a salt thereof, age, body weight, symptom, dosage form, administration method, dosing period and the like, for example, it is generally administered intravenously at about 0.01 - about 1000 mg/kg/day, preferably about 0.01 - about 100 mg/kg/day, more preferably about 0.1 - about 100 mg/kg/day, particularly about 0.1 - about 50 mg/kg/day, specifically about 1.5 - about 30 mg/kg/day, for each of Compound (I) or a salt thereof and drug (II), once a day or in several portions a day per patient (adult, body weight about 60 kg). Needless to say, since the dose varies depending on various conditions as mentioned above, an amount smaller than the aforementioned dose is sometimes sufficient, and administration beyond the range may be sometimes necessary. Drug (II) can be used in any amount as long as the side effects do not pose any problem. The daily dose of drug (II) varies depending on the level of symptoms, age, sex, body weight of the subject of administration, sensitivity difference, administration stage, intervals, properties, formulation, kind of the pharmaceutical preparation, kind of the active ingredient and the like, and is not particularly limited. The dose of the drug is generally, for example, about 0.001 - about 2000 mg, preferably about 0.01 - about 500 mg, more preferably about 0.1 - about 100 mg, per 1 kg body weight of a mammal by oral administration, which amount is generally administered in 1 to 4 portions a day.

### Effect of the Invention

The medicament and pharmaceutical composition of the present invention have D₂ receptor partial agonist effect, 5-HT_{2A} receptor antagonist effect and serotonin uptake inhibitory effect (or serotonin reuptake inhibitory effect).

The D₂ receptor partial agonist effect suppresses dopaminergic (dopamine; DA) neurotransmission when it is enhanced, and accelerates the DAergic neurotransmission when it is lowered and thus has a function to stabilize the DA neurotransmission to a normal state (dopamine system stabilizer). According to this function, excellent clinically improving effect on the DA abnormal neurotransmission (enhancement and lowering), for example, improving effect on positive and negative symptoms, improving effect on cognitive impairment, improving effect on depressive symptom etc. are developed with fewer side effects than D2 antagonist (see Michio Toru: Clinical Psychiatry, vol. 46, pages 855 - 864 (2004), Tetsuro Kikuchi and Tsuyoshi Hirose: Brain Science, vol. 25, pages 579 - 583 (2004), and Harrison, T. S. and Perry, C. M.: Drugs 64: 1715-1736, 2004).

5-HT_{2A} receptor antagonist effect reduces extrapyramidal side effects, develops superior clinical effects, and is effective, for example, for improvement of negative symptoms, improvement of cognitive impairment, improvement of depressive symptom, improvement of insomnia and the like (see Jun Ishigooka and Ken Inada: Japanese Journal of Clinical Psycopharmacology, vol. 4, pages 1653 - 1664 (2001), Mitsukuni Murasaki: Japanese Journal of Clinical Psycopharmacology, vol. 1, pages 5 - 22 (1998), Pullar, I.A. et al.: Eur. J. Pharmacol., 407: 39-46, 2000, and Meltzer, H. Y. et al.: Prog. Neuro-psychopharmacol. Biol. Psychiatry 27: 1159-1172, 2003).

Serotonin uptake inhibitory effect (or serotonin reuptake inhibitory effect) is effective, for example, for improvement of depressive symptom (see Mitsukuni Murasaki: Japanese Journal of Clinical Psycopharmacology, vol. 1, pages 5 - 22 (1998)).

The medicament and pharmaceutical composition of the present invention are excellent in all of these three effects, or remarkably excellent in one or two of these effects.

In addition, some of the medicaments and pharmaceutical compositions of the present invention have α₁ receptor antagonist effect in addition to the above-mentioned effects. The α₁ receptor antagonist effect is effective for improving positive symptoms of schizophrenia (see Svensson, T. H.: Prog. Neuro-psychopharmacol. Biol. Psychiatry 27: 1145-1158, 2003).

Therefore, the medicament and pharmaceutical composition of the present invention have a wide treatment spectrum for and excellent clinical effect on central nervous system diseases.

Accordingly, the medicament and pharmaceutical composition of the present invention are extremely effective for the improvement of various central nervous system disorders including schizophrenia, treatment-resistant, refractory or chronic schizophrenia, emotional disturbance, psychotic disorder, mood disorder, bipolar disorder (e.g., bipolar disorder type I and bipolar disorder type II), mania, depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, acute stress disorder, etc.), somatoform disorder (e.g., hysteria, somatization disorder, conversion disorder, pain disorder, hypochondriasis, etc.), factitious disorder, dissociative disorder, sexual disorder (e.g., sexual dysfunction, sexual desire disorder, sexual arousal disorder, erectile dysfunction, etc.), eating disorder (e.g., anorexia nervosa, bulimia nervosa, etc.), sleep disorder, adjustment disorder, substance-related disorder (e.g., alcohol abuse, alcohol intoxication and drug addiction, stimulant intoxication, narcotism, etc.), anhedonia (e.g., iatrogenic anhedonia, anhedonia of a psychic or mental cause, anhedonia associated with depression, anhedonia associated with schizophrenia, etc.), delirium, cognitive impairment, cognitive impairment associated with Alzheimer's disease, Parkinson's disease, and other neurodegenerative diseases, cognitive impairment caused by Alzheimer's disease, Parkinson's disease and associated neurodegenerative diseases, cognitive impairment in schizophrenia, cognitive impairment caused by treatment-resistant, refractory or chronic schizophrenia, vomiting, motion sickness, obesity, migraine, pain, mental retardation, autistic disorder (autism), Tourette's disorder, tic disorder, attention deficit hyperactivity disorder, conduct disorder, Down's syndrome and the like.

Mental disorders such as mood disorders including depression and major depressive disorder, bipolar disorder, anxiety disorder, schizophrenia and the like belong to an extremely heterogenous disease group. While the critical cause thereof has not been clarified as yet, abnormalities in the central monoaminergic nervous system of serotonin, norepinephrine, dopamine and the like, and abnormalities of various hormones and peptides are said to be involved (Masaharu Kubota et al.: Japanese Journal of Clinical Psychiatry, vol. 29, pages 891 - 899 (2000)). In heterogenous disease group, a wide treatment spectrum can eventually enhance clinical effects, and can secure safety and tolerability. A wide treatment spectrum can be achieved by a combined use of drugs having particularly different action mechanisms. Thus, in the present invention, the effects shown below can be exhibited by combining Compound (I) or a salt thereof and drug (II) selected from a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug and an anti-ADHD drug, which have different action mechanism from that of Compound (I) or a salt thereof, as compared to single administration of Compound (I) or a salt thereof or drug (II).
(1) Oral administration is possible and the dose can be reduced.
(2) Treatment spectrum can be widened, and an effect is also provided for treatment insufficient, or treatment resistant patients, for whom single administration of existing medication fails to show effect.
(3) Duration of treatment can be set shorter, namely, a brief therapy is possible.
(4) Effective dose can be decreased, which leads to superior tolerability and safety, and decreased side effects.
(5) Treatment effect can be sustained.
(6) A synergistic effect can be obtained.
(7) Drug (II) to be combined with Compound (I) or a salt thereof can be selected according to the symptoms (mild, severe, etc.) of the patients.

Moreover, the medicament and pharmaceutical composition of the present invention can reduce the incidence rate of extrapyramidal side effects and akathisia.

### Pharmacological test 1

### Evaluation of antidepressant action in forced swimming test in mice

A forced swimming test is an animal model devised by Porsolt et al. for the evaluation of an antidepressant effect of a drug. When an animal (mouse) is put in a cylindrical water tank (diameter about 9 cm, height 25 cm) filled with water (water temperature 23 - 25°C) up to about 10 cm, it shows immobility a little while later. When an antidepressant is administered in advance, the immobility time is shortened. The shortened immobility time is used as an index of an antidepressant action to be evaluated. This test method is widely used as an experimental animal model reflecting the clinical antidepressant effect.

In this test, the action of a combined use of 7-[4-(4-benzo[b]thiophen-4-yl-piperazin-1-yl)butoxy]-1H-quinolin-2-one (Compound (I)) and 9 kinds of antidepressants (serotonin reuptake inhibitors, or serotonin and norepinephrine reuptake inhibitors) on the immobility time was compared.

As the animal, ddY male mice (5 to 6-week-old) were used. Various antidepressants and Compound (I) or each vehicle were administered to the mice before the start of the test, the mice were placed in a cylindrical water tank, and immobility time during 2 min to 4 min after the start of swimming was measured by a behavior analysis apparatus with an infrared sensor (SCANET; Melquest Co., Ltd.).

Compound (I) was dissolved in 1% lactic acid-physiological saline, and various antidepressants were suspended or dissolved in 5% gum arabic-distilled water and used. Various antidepressants (fluoxetine (75 mg/kg), escitalopram (60 mg/kg), paroxetine (10 mg/kg), sertraline (15 mg/kg), venlafaxine (15 mg/kg), milnacipran (30 mg/kg), fluvoxamine (75 mg/kg)) were orally administered 60 min before the start of the test, duloxetine (20 mg/kg) and desvenlafaxine (20 mg/kg) were orally administered 30 min before the start of the test, and Compound (I) (0.001 or 0.003 mg/kg) was intraperitoneally administered 15 min before the start of the test. The results are shown in Tables 1-1 and 1-2.

**[Table 1-1]**

| Compound (I) | dose (mg/kg) | drug (II) | dose (mg/kg) | number | immobility time (3-6 min after start of swimming, unit: sec) | |
|---|---|---|---|---|---|---|
| | | | | | (average ± standard error) | relative to control (%) |
| vehicle 1 | 0 | vehicle 2 | 0 | 10 | 226.5±4.4 | 100 |
| Compound (I) | 0.003 | vehicle 2 | 0 | 10 | 227.4±2.7 | 100 |
| vehicle 1 | 0 | fluoxetine | 75 | 10 | 209.3±10.0 | 92 |
| Compound (I) | 0.003 | fluoxetine | 75 | 10 | 180.6±8.4 *** | 80 |
| vehicle 1 | 0 | vehicle 2 | 0 | 10 | 232.2±2.8 | 100 |
| Compound (I) | 0.003 | vehicle 2 | 0 | 10 | 223.1±4.3 | 96 |
| vehicle 1 | 0 | escitalopram | 60 | 10 | 221.5±7.7 | 95 |
| Compound (I) | 0.003 | escitalopram | 60 | 10 | 208.7±8.6 * | 90 |
| vehicle 1 | 0 | vehicle 2 | 0 | 10 | 223.0±3.9 | 100 |
| Compound (I) | 0.003 | vehicle 2 | 0 | 10 | 220.7±6.0 | 98 |
| vehicle 1 | 0 | paroxetine | 10 | 10 | 217.3±4.7 | 98 |
| Compound (I) | 0.003 | paroxetine | 10 | 10 | 189.5±13.7 * | 87 |
| vehicle 1 | 0 | vehicle 2 | 0 | 7 | 222.6±5.8 | 100 |
| Compound (I) | 0.003 | vehicle 2 | 0 | 7 | 194.7±7.4 | 87 |
| vehicle 1 | 0 | sertraline | 15 | 7 | 203.6±6.3 | 91 |
| Compound (I) | 0.003 | sertraline | 15 | 7 | 172.2±7.7 *** | 77 |
| vehicle 1 | 0 | vehicle 2 | 0 | 10 | 231.9±1.8 | 100 |
| Compound (I) | 0.001 | vehicle 2 | 0 | 10 | 228.8±3.2 | 99 |
| vehicle 1 | 0 | venlafaxine | 15 | 10 | 223.6±4.3 | 96 |
| Compound (I) | 0.001 | venlafaxine | 15 | 10 | 198.1±15.4 * | 85 |
| vehicle 1 | 0 | vehicle 2 | 0 | 10 | 223.9±3.5 | 100 |
| Compound (I) | 0.003 | vehicle 2 | 0 | 10 | 226.8±4.5 | 101 |
| vehicle 1 | 0 | milnacipran | 30 | 10 | 217.9±6.9 | 97 |
| Compound (I) | 0.003 | milnacipran | 30 | 10 | 200.4±9.7 * | 89 |

**[Table 1-2]**

| Compound (I) | dose (mg/kg) | drug (II) | dose (mg/kg) | number | immobility time (3-6 min after start of swimming, unit: sec) | |
|---|---|---|---|---|---|---|
| | | | | | (average ± standard error) | relative to control (%) |
| vehicle 1 | 0 | vehicle 2 | 0 | 7 | 216.8±7.7 | 100 |
| Compound (I) | 0.003 | vehicle 2 | 0 | 7 | 213.5±9.5 | 98 |
| vehicle 1 | 0 | duloxetine | 20 | 7 | 205.6±12.3 | 95 |
| Compound (I) | 0.003 | duloxetine | 20 | 7 | 170.9±17.0 * | 79 |
| vehicle 1 | 0 | vehicle 2 | 0 | 10 | 213.4±7.8 | 100 |
| Compound (I) | 0.003 | vehicle 2 | 0 | 10 | 214.8±5.1 | 100 |
| vehicle 1 | 0 | fluvoxamine | 75 | 10 | 203.3±6.2 | 95 |
| Compound (I) | 0.003 | fluvoxamine | 75 | 10 | 179.5±9.3 ** | 84 |
| vehicle 1 | 0 | vehicle 2 | 0 | 8 | 227.8±1.7 | 100 |
| Compound (I) | 0.003 | vehicle 2 | 0 | 8 | 218.3±8.4 | 96 |
| vehicle 1 | 0 | desvenlafaxin | 20 | 8 | 215.7±5.9 | 95 |
| Compound (I) | 0.003 | desvenlafaxin | 20 | 8 | 181.9±10.5 *** | 80 |

| | | | | | | |
|---|---|---|---|---|---|---|
| vehicle for Compound (I) (vehicle 1: 1% lactic acid-physiological saline) vehicle for each antidepressant (vehicle 2: 5% gum arabic-distilled water) *p<0.05, **p<0.01, ***p<0.001; comparison with each vehicle 1 + vehicle 2 group (after the analysis of variance, one-way layout Dunnett's test). | | | | | | |

When Compound (I) was used in combination with fluoxetine, escitalopram, paroxetine, sertraline, venlafaxine, milnacipran, duloxetine, fluvoxamine or desvenlafaxine, a remarkably high immobility time shortening action (antidepressant action) was shown as compared to single use of Compound (I), fluoxetine, escitalopram, paroxetine, sertraline, venlafaxine, milnacipran, duloxetine, fluvoxamine or desvenlafaxine.

### Pharmacological test 2

### Tail suspension test

A tail suspension test was devised by Steru et al. (Psychopharmacology (Berl). 1985; 85(3): 367-70.). A mouse suspended by its tail shows periods of agitation and immobility. The antidepressant activity of a test compound can be detected with shortened immobility time as an index. This test is widely used as an experimental animal model for predicting the antidepressant activity in clinical settings. A mouse is suspended by its tail, and immobility time is measured for a certain period of time, for example, 6 min, by a tail suspension test apparatus. By this test method, the medicament of the present invention comprising Compound (I) and drug (II) in combination can be confirmed to show an unpredictably superior antidepressant action, and emotional disturbance improving effect.

### Pharmacological test 3

### Motor activity enhancement model

An experiment is performed using a partly-modified bipolar disorder model by Frey et al. (Frey, B.N. et al.: Life Sci., 79: 281-286, 2006). Since administration of amphetamine or methamphetamine to mouse markedly increases the motor activity of the mouse, an increase of motor activity of the mouse is considered to be a bipolar disorder model. Motor activity is measured for a certain period of time, for example, 1 hr, after methamphetamine administration to the mouse, by a motor activity monitoring apparatus. By this test method, the medicament of the present invention comprising Compound (I) and drug (II) in combination can be confirmed to show an unpredictably superior bipolar disorder improving effect.

### Pharmacological test 4

### Enhancing effect on mouse marble-burying behavior by combined use of Compound (I) and antianxiety drug or antidepressant

Marble-burying behavior test is a behavioral evaluation test utilizing the behavior of a mouse to bury marbles on the bedding. Since this characteristic behavior of burying harmless glass marbles in the bedding reflects the anxiety state of the animal, and is apparently similar to a compulsive act of obsessive-compulsive disorder patient, which is repeated with the recognition of irrationality, it is particularly considered a model of obsessive-compulsive disorder ^{1) ,2) ,3) ,4)}. It has been reported that a general antianxiety drug, a selective serotonin reuptake inhibitor (SSRI), and a selective serotonin and norepinephrine reuptake inhibitor (SNRI) suppress the burying behavior without suppressing mortor activity in this test system.

In this test, an enhancing effect of a combined use of an antianxiety drug or an antidepressant, which is considered to be effective for obsessive-compulsive disorder, and Compound (I) on the antianxiety or obsessive-compulsive disorder-improving action reflected in the marble-burying behavior was considered using this model.

As the animal, male ddY mice (6- to 7-week-old) were used. In the test, a plastic cage (26 x 32 x 17 cm) was filled with chips to about 5 cm deep in a soundproof room, and 25 marbles (diameter 15 mm, weight about 6 g) were placed thereon at equal distances. Each mouse was orally administered with Compound (I) simultaneously with an antianxiety drug or an antidepressant or a vehicle thereof, and 30 min later, each mouse was placed in a cage containing the marbles. 30 min later, the animal was taken out from the cage, and the number of marbles covered with chips by 2/3 or more was visually counted.

Compound (I) (0.1 mg/kg) and various antidepressants (fluoxetine (10 mg/kg), paroxetine (10 mg/kg), sertraline (10 mg/kg), fluvoxamine (15 mg/kg)) were suspended in 5% gum arabic-distilled water, and diazepam (antianxiety drug, 1 mg/kg) was diluted with physiological saline and used. All drugs and vehicles thereof were orally administered 30 min before the start of the test. The results are shown in Table 2.

### References

1) Njung'e K, Handley SL. Evaluation of Marble-Burying Behavior as a Model of Anxiety. Pharmacol Biochem Behav. 1991; 38(1):63-67.
2) Ichimaru Y, et al. 5HT1A-receptor subtype mediates the effect of fluvoxamine, a selective serotonin reuptake inhibitor, on marble-burying behavior. Jpn J Pharmacol. 1995;68(1):65-70.
3) Yamanaka K, et al. Effect of repeated dosing of sertraline on marble-burying behavior in mice. Jpn J Neuropsychopharmacol. 1997;19(6):387-393.
4) Sugimoto Y, et al. Effects of the Serotonin and Noradrenaline Reuptake Inhibitor (SNRI) Milnacipran on Marble Burying Behavior in Mice. Biol. Pharm. Bull. 2007;30(12):2399-2401.

**[Table 2]**

| Compound (I) | dose (mg/kg) | drug (II) | dose (mg/kg) | number | number of marbles buried | |
|---|---|---|---|---|---|---|
| | | | | | (average ± standard error) | relative to control (%) |
| vehicle 1 | 0 | vehicle 2 | 0 | 10 | 15.0±2.2 | 100 |
| Compound (I) | 0.1 | vehicle 2 | 0 | 10 | 14.4±2.0 | 96 |
| vehicle 1 | 0 | diazepam | 1 | 10 | 12.8±2.0 | 85 |
| Compound (I) | 0.1 | diazepam | 1 | 10 | 5.5±2.0 *** | 37 |
| vehicle 1 | 0 | vehicle 1 | 0 | 10 | 17.6±1.7 | 100 |
| Compound (I) | 0.1 | vehicle 1 | 0 | 10 | 14.4±2.0 | 82 |
| vehicle 1 | 0 | fluoxetine | 10 | 10 | 17.3±1.5 | 98 |
| Compound (I) | 0.1 | fluoxetine | 10 | 10 | 7.8±1.4 *** | 44 |
| vehicle 1 | 0 | vehicle 1 | 0 | 10 | 12.6±2.3 | 100 |
| Compound (I) | 0.1 | vehicle 1 | 0 | 10 | 11.3±1.5 | 90 |
| vehicle 1 | 0 | paroxetine | 10 | 10 | 8.5±2.0 | 60 |
| Compound (I) | 0.1 | paroxetine | 10 | 10 | 5.2±1.4 * | 47 |
| vehicle 1 | 0 | vehicle 1 | 0 | 10 | 13.4±1.8 | 100 |
| Compound (I) | 0.1 | vehicle 1 | 0 | 10 | 12.2±2.2 | 91 |
| vehicle 1 | 0 | sertraline | 10 | 10 | 7.5±0.7 * | 56 |
| Compound (I) | 0.1 | sertraline | 10 | 10 | 3.3±1.1 ** | 25 |
| vehicle 1 | 0 | vehicle 1 | 0 | 10 | 16.2±1.1 | 100 |
| Compound (I) | 0.1 | vehicle 1 | 0 | 10 | 14.0±1.6 | 86 |
| vehicle 1 | 0 | fluvoxamine | 15 | 10 | 13.5±2.1 | 83 |
| Compound (I) | 0.1 | fluvoxamine | 15 | 10 | 3.8±1.1 *** | 23 |

| | | | | | | |
|---|---|---|---|---|---|---|
| vehicle for Compound (I) and each antidepressant (vehicle 1: 5% gum arabic-distilled water) vehicle for diazepam (vehicle 2: physiological saline) *p<0.05, **p<0.01, ***p<0.001; comparison with each vehicle + vehicle group (after the analysis of variance, one-way layout Dunnett's test). | | | | | | |

### Pharmacological test 5

### Evaluation of Compound (I) on locomotor activity of SHR (Spontaneous Hypertensive Rats) in a novel environment

It is known that, after maturation, SHR becomes spontaneously hypertensive, shows low dopaminergic neuronal activity in the striatum, and shows high motor activity in a novel environment at normal times, as compared to WKY-Kyoto rats, to which species the SHR belongs. This state is considered to reflect the hyperactivity of ADHD, and it has been reported that drugs considered to be effective for human ADHD, such as methylphenidate and amphetamine, decrease the increased locomotor activity more in this model than in WKY-Kyoto rats (Sagvolden T, et al. Biol Psychiatry. 2005;57(11):1239-47).

In this test, to evaluate the treatment effect of a combined use of Compound (I) and an anti-ADHD drug in ADHD, an effect on increased motor activity of SHR rats in a novel environment is examined. In this test, using 12- to 14-week-old SHR rats, a drug to be evaluated and a vehicle thereof are administered and, after a given time, the rats are placed in a novel environment, e.g., motor activity monitoring apparatus, and then spontaneous motor activity for 1 hr thereafter is measured using a motor activity monitoring apparatus with an infrared sensor (Supermex: Muromachi Kikai Co., Ltd.). For example, the treatment effect on ADHD can be evaluated by using Compound (I) and a representative anti-ADHD drug in combination, and subjecting them to this test.

### Pharmacological test 6

Multicenter, randomized, double-blind, placebo-controlled study to examine safety and treatment effect of Compound (I) when Compound (I) is added on psychostimulant in adult ADHD patients showing insufficient treatment effect of psychostimulant

### Test method

### Phase A (single blind prospective treatment phase)

18- to 55-year-old patients diagnosed with ADHD based on DSM-IV-TR were registered to a prospective treatment phase (Phase A). In Phase A, the subjects took a psychostimulant approved in the USA and selected by the investigators, or placebo, by single blind (investigators alone know the kind of the drug, and the subject does not know the kind of the drug). Phase B (double-blind randomized phase)

On the final day of Phase A, the subjects showing an insufficient treatment effect were transferred to a 6-week double-blind randomized phase (Phase B), and underwent a total of 11 weeks of treatment including Phase A and Phase B. The subjects were assigned to either of the following groups by double-blind.
- placebo + psychostimulant
- Compound (I) 0.25-2 mg/day + psychostimulant

### Evaluation method

The primary endpoint was evaluation of safety and tolerance of Compound (I) by comparing the improvement of ADHD between Compound (I) group and placebo group from the final day of Phase A (week 5, hospital visit) to the final day of Phase B (week 11, hospital visit).

For evaluation of the side-effects, last observation carried forward (LOCF) of Simpson-Angus Scale (SAS), AIMS (Abnormal Involuntary Movement) and Barnes Akathisia Rating Scale (BARS) were used for both extrapyramidal side effects and akathisia, in addition to the laboratory test including adverse event, physical findings, vital signs, 12-induced ECG, serum prolactin, and measurement of body weight.

Formulation examples comprising Compound (I) are described below, which are not to be construed as limitative.

For treatment of central nervous system disorders such as schizophrenia, drug dosage forms that are effective for a long time (at least one week; more preferably 2, 3, or 4 weeks; still more preferably more than 6 weeks) are useful because patient compliance is improved, and probability of relapse can be reduced.

However, no such long-term effective dosage form is known for Compound (I), or salts thereof.

An object of the present invention is to provide a composition or a dosage form that enables the Compound (I) or salts thereof to be effective for a long time; and a process for preparing the same.

The compositions of the invention are believed to have advantages such as (but not limited to) one or more of the following.

The compositions of the invention allow Compound (I) to be administered less frequently. Any reduction in dosing frequency is thought to bring material improvements in patient convenience and compliance.

Reduction in dosing frequency offers significant pharmacoeconomic advantages over the current dosing regimen by reducing the indirect human cost of drug treatment (e.g., by reducing the time required by medical practitioners for supervised drug administration).

The compositions of the invention also provide a once-daily dosage regimen in which the release of Compound (I) is controlled. It is thought that this could reduce the adverse event profile compared to currently available once-daily dosage regimens, and/or provide a more efficient once-daily dose regimen.

Unless otherwise indicated herein, the term "Compound (I)" refers to 7-[4-(4-benzo[b]thiophen-4-yl-piperazin-1-yl)butoxy]-1H-quinolin-2-one, its pharmaceutically acceptable salts, and mixtures thereof. "Pharmaceutically acceptable salts" includes derivatives of Compound (I), wherein Compound (I) is modified by making non-toxic acid or base salts thereof; and further refers to pharmaceutically acceptable solvates of such salts. Examples of pharmaceutically acceptable salts include, but are not limited to, mineral or organic acid salts of the amine functionality of Compound (I). The pharmaceutically acceptable salts include non-toxic salts and the quaternary ammonium salts of Compound (I) formed, for example, from organic and inorganic acids. Such salts include those derived from inorganic acid, such as hydrochloric, hydrobromic, hydroiodic, sulfuric, phosphoric, and nitric; metal salts, such as sodium salt, potassium salt, and cesium salt; alkaline earth metal salts, such as calcium salt and magnesium salt; and combinations of the foregoing. Pharmaceutically acceptable organic salts include salts prepared from organic acids such as acetic, trifluoroacetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethanedisulfonic, oxalic, isethionic, and HO₂C-(CH₂)ₙ-CO₂H (wherein n = 0-4); and salts prepared from amino acids such as arginate, asparginate and glutamate. Preferred pharmaceutically acceptable organic salts include salts prepared from organic acids such as acetic, trifluoroacetic, propionic, succinic, glycolic, stearic, lactic, malic, tartaric, citric, ascorbic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethanedisulfonic, oxalic, isethionic, and HO₂C-(CH₂)ₙ-CO₂H (wherein n = 0-4); and salts prepared from amino acids such as arginate, asparginate and glutamate. The term "pharmaceutically acceptable salts" also includes mixtures of any of the foregoing derivatives of Compound (I).

By the term "orally deliverable", we include the meaning suitable for oral, including peroral and intra-oral (e.g., sublingual or buccal) administration. Preferably, the compositions of the invention are designed for peroral administration to a patient, i.e., by swallowing (e.g., eating or drinking).

By the term "controlled release", we include the meaning that after administration, release of the Compound (I) is controlled so that a dosage regimen in which Compound (I) can be administered, for example, less frequently than once daily, can be provided (however, improved release profiles for once-daily administration are also included in this regard). This may include delaying and/or prolonging and/or sustaining the release of Compound (I), so that the time between doses of Compound (I) can be increased. Such delayed/prolonged/sustained release may also be accompanied by a higher single dose of Compound (I) in the compositions of the invention.

The compositions of the invention are suitable for a controlled-release, once-daily (OD) dose regimen, and dose regimens less frequent than OD. By dose regimens less frequent than OD, we include once every 2, 3, 4, 5 or 6 days; thrice weekly; twice weekly (TW); once weekly (OW); and combinations thereof. A preferred group of dose regimens are OD; once every 2 days (i.e., every other day); TW and OW, for example, once every 2 days; and TW and OW.

The controlled-release characteristics of the compositions of the invention may be defined in relation to their *in vitro* or *in vivo* release profiles; or related values, such as Cₘₐₓ, Tₘₐₓ and AUC, as described in more detail below.

For example, the compositions of the invention may exhibit an *in vitro* release profile wherein, on average, no more than about 60% of the Compound (I), preferably no more than about 50%, more preferably no more than about 40%, is dissolved within 3 hours after placement in a standard dissolution test.

Unless otherwise indicated, as used herein, the term "standard dissolution test" means a test conducted according to the "Paddle Method" at 100 rpm in 900 ml of a dissolution medium by an aqueous physiological aspect with a pH range between 1 and 7 at 37°C, as described in the United States Pharmacopoeia; or other test conditions substantially equivalent thereto, e.g., 0.1 M hydrochloric acid and pH 4.0 phosphate buffer.

As noted above, the subject invention concerns providing dosage regimens with either low-frequency dosing, or controlled delivery of the daily dose. Compositions having the *in vitro* release profile defined above may be suitable for both OD administration and dose regimens requiring even less-frequent administration of the drug-containing composition than OD, as explained in more detail below.

To avoid confusion, by the phrase "dose regimens requiring even less-frequent administration of the drug containing composition than OD", as used herein in relation to the compositions of the invention having the controlled-release characteristics described herein (e.g., an *in vitro* release profile), we include once every 2, 3, 4, 5 or 6 days; thrice weekly; twice weekly (TW); once weekly (OW); and combinations thereof.

The compositions of the invention that may be suitable for OD administration may typically exhibit an *in vitro* release profile wherein, on average, about 10 to about 50%, such as about 15 to about 45%; for example, about 15 to about 30%, of the Compound (I) is dissolved within 3 hours after placement in a standard dissolution test.

The compositions of the invention that may be suitable for dose regimens requiring even less-frequent administration of the drug-containing composition than OD may typically exhibit an *in vitro* release profile wherein, on average, about 2 to about 40% (e.g., about 2 to about 30 or 35%), such as about 5 to about 25%; for example, about 10 to about 20%, of the Compound (I) is dissolved within 3 hours after placement in a standard dissolution test.

The compositions of the invention that may be suitable for OD administration may typically exhibit an *in vitro* release profile wherein, on average, about 25 to about 100%, such as about 30 to about 100%; for example, about 40 to about 100%, or about 50 to about 100%, of the Compound (I) is dissolved within 8 hours after placement in a standard dissolution test.

The compositions of the invention that may be suitable for dose regimens requiring even less-frequent administration of the drug-containing composition than OD may typically exhibit an *in vitro* release profile wherein, on average, no more than about 70% of the Compound (I), preferably no more than about 60%, more preferably no more than about 50%; for example, no more than about 40%, is dissolved within 8 hours after placement in a standard dissolution test. Typically, such compositions exhibit an *in vitro* release profile wherein, on average, about 10 to about 65%, such as about 15 to about 55%; for example, about 20 to about 45%, of the Compound (I) is dissolved within 8 hours after placement in a standard dissolution test.

The compositions of the invention may exhibit an in *vitro* dissolution rate after placement in a standard dissolution test wherein:
about 2 to about 50% of the Compound (I) is released after 2 hours;
about 5 to about 80% of the Compound (I) is released after 4 hours;
25% or more of the Compound (I) is released after 8 hours; and 40% or more of the Compound (I) is released after 12 hours.

Preferably, the *in vitro* release rate is independent of pH between 1 and 7.

Compositions having the *in vitro* release profile defined above may be suitable for both OD administration and dose regimens requiring even less-frequent administration of the drug-containing composition than OD, as explained in more detail below.

The compositions of the invention that may be suitable for OD administration may typically exhibit an *in vitro* dissolution rate after placement in a standard dissolution test wherein:
about 5 to about 40% (e.g., 10 to 30%) of the Compound (I) is released after 2 hours;
about 15 to about 70% (e.g., 20 to 50%) of the Compound (I) is released after 4 hours; and
50% or more (e.g., 60% or more) of the Compound (I) is released after 8 hours.

Preferably, the *in vitro* release rate is independent of pH between 1 and 7.

The compositions of the invention that may be suitable for dose regimens requiring even less-frequent administration of the drug-containing composition than OD may typically exhibit an *in vitro* dissolution rate after placement in a standard dissolution test, wherein:
about 2 to about 35%, such as about 2 to about 25% (e.g., 5 to 15%) of the Compound (I) is released after 2 hours;
about 5 to about 50% (e.g., 10 to 40) of the Compound (I) is released after 4 hours;
about 25 to about 80% (e.g., 30 to 60) of the Compound (I) is released after 8 hours; and
40% or more (e.g., 50% or more) of the Compound (I) is released after 12 hours.

Preferably, the *in vitro* release rate is independent of pH between 1 and 7.

The compositions of the invention may exhibit an *in vivo* Compound (I) plasma absorption profile following single-dose oral administration, wherein the time for 50% of the Compound (I) to be absorbed into the plasma (of a human or animal patient) is at least 2 hours, preferably at least 3 hours, more preferably at least 4 hours (e.g., at least about 5 or 6 hours).

The phrase "Compound (I) plasma absorption profile" is intended to refer to the plasma concentration of Compound (I) over time following administration to a human or animal patient. As known to those skilled in the art, the plasma absorption profile may be measured by deconvolution of continuous-release pharmacokinetics versus an immediate-release reference.

Compositions having the *in vivo* release profile defined above may be suitable for both OD administration and dose regimens requiring even less-frequent administration of the drug-containing composition than OD, as explained in more detail below.

The compositions of the invention that may be suitable for OD administration may typically exhibit an *in vivo* Compound (I) plasma absorption profile following single-dose oral administration, wherein the time for 50% of the Compound (I) to be absorbed into the plasma is about 2 to about 12 hours, such as about 3 to about 10 hours; for example, about 4 to about 9 hours, or about 5 to about 7 hours (e.g., about 6 hours).

The compositions of the invention that may be suitable for dose regimens requiring even less-frequent administration of the drug-containing composition than OD may typically exhibit an *in vivo* Compound (I) plasma absorption profile following single-dose oral administration, wherein the time for 50% of the Compound (I) to be absorbed into the plasma is about 6 to about 24 hours, such as about 7 to about 20 hours; for example, about 8 to about 18 hours, or about 10 to about 16 hours.

The compositions of the invention may also be defined in terms of the amount of Compound (I) that is released from the compositions *in vivo* at specified periods of time following oral administration. For example, compositions of the invention may typically exhibit a release profile wherein: about 2 to about 50% of the Compound (I) is released within 2 hours following administration;
about 5 to about 80% of the Compound (I) is released within 4 hours following administration;
25% or more of the Compound (I) is released within 8 hours following administration; and
40% or more of the Compound (I) is released within 12 hours following administration.

Compositions having the *in vivo* release profile defined above may be suitable for both OD administration, and for dose regimens requiring even less frequent administration of the drug-containing composition than OD, as explained in more detail below.

The compositions of the invention that may be suitable for OD administration may typically exhibit an *in vivo* Compound (I) plasma absorption profile wherein:
about 5 to about 40% (e.g., 10 to 30%) of the Compound (I) is released within 2 hours following administration;
about 15 to about 70% (e.g., 20 to 50%) of the Compound (I) is released within 4 hours following administration; and
50% or more (e.g., 60% or more) of the Compound (I) is released within 8 hours following administration.

The compositions of the invention that may be suitable for dose regimens requiring even less-frequent administration of the drug-containing composition than OD typically exhibit an *in vivo* Compound (I) plasma absorption profile wherein: about 2 to about 35%, such about 2 to about 25% (e.g., 5 to 15%), of the Compound (I) is released within 2 hours following administration;
about 5 to about 50% (e.g., 10 to 40%) by weight of the Compound (I) is released within 4 hours following administration;
about 25 to about 80% (e.g., 30 to 60%) of the Compound (I) is released within 8 hours following administration; and
40% or more (e.g., 50% or more) of the Compound (I) is released within 12 hours following administration.

The controlled-release characteristics of the compositions of the invention that may be suitable for OD administration may be defined in relation to the peak plasma concentration (Cₘₐₓ) value of Compound (I) when administered to human or animal patients. For example, the compositions of the invention that may be suitable for OD administration typically exhibit a Compound (I) Cₘₐₓ value following oral administration of about 10 to about 99%, such as about 20 to about 80%; for example, about 25 to about 70% (e.g., about 30 to about 60%) of the Cₘₐₓ value achieved using a conventional immediate-release (IR) dosage form of Compound (I) when administered orally at an identical dose.

The phrase "conventional immediate-release (IR) dosage form of Compound (I)" includes the meaning that the dosage form releases substantially all of the Compound (I) contained therein immediately; for example, within 30 minutes of administration. In other words, such IR dosage forms typically have substantially no component that acts to delay and/or prolong and/or sustain the release of Compound (I).

The controlled-release characteristics of the compositions of the invention that may be suitable for OD administration may be defined by the ratio of the peak plasma concentration (Cₘₐₓ) of Compound (I) to the plasma concentration of Compound (I) 24 hours following administration (C₂₄) when administered to human or animal patients, and prior to the administration of any further doses. The compositions of the invention typically exhibit a Cₘₐₓ to C₂₄ ratio, preferably under steady-state conditions, that is less than about 3:1, preferably less than about 2:1, more preferably less than about 1.5:1; such as 1.1:1 to about 1.5:1 (e.g., about 1:1).

The compositions of the invention may exhibit one or more of the controlled-release profiles defined above.

The compositions of the invention comprise a therapeutically effective amount of Compound (I) and at least one pharmaceutically acceptable excipient. In order to achieve one or more of the controlled-release profiles described above, the therapeutically effective amount of Compound (I) may be formulated in numerous different ways, including, but not limited to, diffusion-controlled formulations (such as wax matrices or pellets), dissolution-controlled formulations (such as press-coated formulations), dissolution/diffusion-controlled formulations, easily administrable formulations (such as chewable, fast-dissolving, sprinkle or taste-masked formulations), enteric-coated formulations, osmotic pump technology formulations, tamper-resistant formulations, erosion-controlled formulations, ion exchange resins, and combinations of the foregoing. The above formulations will be described in more detail below.

The formulations described herein for the compositions of the invention are designed primarily for oral administration. Suitable oral dosage forms include, but are not limited to, capsules, tablets, liquids, powders, granules, suspensions, matrices, microspheres, seeds, pellets and/or beads of the foregoing formulations. Combinations of these dosage forms may also be used in the invention. For example, an oral dosage form containing Compound (I) may be in the form of microtablets enclosed inside a capsule, e.g., a hydroxypropylmethylcellulose (HPMC) capsule or a gelatin capsule. Any suitable gelatin capsule may be used; for example, the hard gelatin capsule known as CAPSUGEL may be used.

The solid oral dosage forms described above may typically utilize drug substances that may have an average particle size of greater than 100 nm, preferably greater than 500 nm, 1000 nm or 2000 nm (e.g., greater than 2500 nm).

The compositions of the invention may be diffusion-controlled formulations. By the term "diffusion-controlled formulations", we include formulations in which diffusion of dissolved Compound (I) from the formulation has a significant role in the rate of controlled release of Compound (I) from that formulation. However, dissolution processes may also be involved. Typical diffusion-controlled formulations include so-called "reservoir systems", in which a core of Compound (I) is coated with a polymer (typically a water-insoluble polymer); and so-called "matrix systems", in which the Compound (I) is dispersed throughout a matrix (e.g., a swellable matrix), which may optionally be coated. In either system, flow and egress of the dissolved drug is controlled so as to achieve one or more of the release profiles defined above.

The compositions of the invention may be based on matrix technology. In this technology, Compound (I) is embedded in an excipient that makes a non-disintegrating core called a matrix. Diffusion of (dissolved) Compound (I) occurs through the core.

Preferably, the controlled-release compositions of the invention are formulated so there is at least some time delay before significant plasma concentrations of Compound (I) are achieved. In other words, the compositions of the invention may have a delayed- and/or sustained- and/or prolonged-release component. Such compositions may avoid an initial burst of Compound (I), or may be formulated so that release of Compound (I) in a particular part of the gastrointestinal tract (e.g., the stomach) is retarded. This may be useful for minimizing the adverse event profiles associated with Compound (I).

Enteric coated formulations, which may protect the stomach against any irritant effects of Compound (I), are also desirable. Such formulations can be coated with a composition that is non-toxic and includes a pharmaceutically acceptable enteric polymer that is predominantly soluble in the intestinal fluid, but substantially insoluble in the gastric juices.

Typically, the compositions of the invention extend the Compound (I) release by, e.g., several hours, compared to Compound (I) release in the known immediate-release dosage form.

The compositions of the invention may comprise a release-retarding material. The release-retarding material can be, for example, in the form of a matrix or a coating. The compositions of the invention may comprise, for example, a particle of Compound (I) that is combined with a release-retarding material. The release-retarding material is typically a material that permits release of Compound (I) at a sustained rate in an aqueous medium. The release-retarding material can be selectively chosen so as to achieve, in combination with the other stated properties, a desired release rate.

Release-retarding materials may be hydrophilic and/or hydrophobic polymers and/or materials. Suitable release-retarding materials include, but are not limited to, acrylic polymers, alkylcellulose, shellac, zein, hydrogenated vegetable oil, hydrogenated caster oil, and combinations comprising one or more of the foregoing materials. The compositions of the invention typically may contain between about 1% and about 80% (by weight) of the release-retarding material.

Suitable acrylic polymers include, for example, acrylic and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid anhydride), methyl methacrylate, polymethacrylate, poly(methyl methacrylate) copolymer, polyacrylamide, aminoalkyl methacrylate copolymer, glycidyl methacrylate copolymers, and combinations comprising one or more of the foregoing polymers.

Suitable alkylcelluloses include, for example, ethylcellulose. Those skilled in the art will appreciate that other cellulosic polymers, including other alkyl cellulosic polymers, can be substituted for part or all of the ethylcellulose.

Other suitable hydrophobic materials are typically water-insoluble and may have a melting point of about 30°C to about 200°C, preferably about 45°C to about 90°C. The hydrophobic material may include neutral or synthetic waxes, fatty alcohols (such as lauryl, myristyl, stearyl, cetyl or preferably cetostearyl alcohol), fatty acids, including fatty acid esters, fatty acid glycerides (mono-, di-, and triglycerides), hydrogenated fats, hydrocarbons, hardened oils or fats (e.g., hardened rapeseed oil, caster oil, beef tallow, palm oil, soya bean oil), waxes, stearic acid, stearyl alcohol, polyethylene glycol, hydrophobic and hydrophilic materials having hydrocarbon backbones, and combinations comprising one or more of the foregoing materials.

Suitable waxes include beeswax, glycowax, castor wax, carnauba wax and wax-like substances, e.g., materials that are normally solid at room temperature and have a melting point of about 30°C to about 100°C, and combinations comprising two or more of the foregoing waxes.

The release-retarding material also may comprise digestible, long chain (e.g., C₈-C₅₀, preferably C₁₂-C₄₀), substituted or unsubstituted hydrocarbons, such as fatty acids, fatty alcohols, glyceryl esters of fatty acids, mineral and vegetable oils, waxes, and combinations comprising one or more of the foregoing materials. Hydrocarbons having a melting point of about 25°C to about 90°C may be used. The compositions of the invention may contain up to about 60% by weight of at least one digestible, long chain hydrocarbon and/or up to 60% by weight of at least one polyalkylene glycol.

The release-retarding material also may comprise polylactic acid, polyglycolic acid, or a co-polymer of lactic acid and glycolic acid. The release-retarding material optionally includes other additives, such as an erosion-promoting agent (e.g., starch and gums) and/or a semi-permeable polymer.

Release-modifying agents, which affect the release properties of the composition, may optionally be used in the compositions of the invention. The release-modifying agent may, for example, function as a pore-former. Typically, a pore-former creates channels that facilitate (e.g., accelerate) drug release. The pore-former can be organic or inorganic, and may include materials that can be dissolved, extracted or leached from the coating in the environment of use. The pore-former can comprise one or more hydrophilic polymers, such as hydroxypropylmethylcellulose, lactose, metal stearates (e.g., alkali metal stearates such as magnesium stearate), polycarbonates (linear polyesters of carbonic acid in which carbonate groups reoccur in the polymer chain), and combinations comprising two or more of the foregoing release-modifying agents.

The release-retarding material can also include an exit means comprising at least one passageway, orifice, or the like. The passageway can have any shape, such as round, triangular, square or elliptical. Such an exit means may be used in osmotic pump formulations, which are described in more detail herein.

In addition to the above ingredients, the compositions of the invention may also contain suitable quantities of other materials, e.g., diluents, lubricants, binders, granulating aids, colorants, flavorants and glidants that are conventional in the pharmaceutical art.

Examples of suitable lubricants include stearic acid, magnesium stearate, glyceryl behenate, talc, and mineral oil (in PEG). Examples of suitable binders include water-soluble polymers, such as modified starch, gelatin, polyvinylpyrrolidone, polyvinyl alcohol, etc. Examples of suitable fillers include lactose and microcrystalline cellulose. An example of a glidant is silicon dioxide.

The compositions of the invention may include one or more substrates comprising Compound (I). Such substrates may be coated with a sustained- and/or delayed- and/or prolonged-release coating comprising a release-retarding material. Such compositions may be used in a multiparticulate system, such as beads, ion-exchange resin beads, spheroids, microspheres, seeds, pellets, matrices, granules, and other multiparticulate systems, in order to obtain the desired controlled release of Compound (I). The multiparticulate system can be presented in a capsule or other suitable unit dosage form, such as a tablet or a sachet.

In certain cases, more than one multiparticulate system may be used, each exhibiting different characteristics, such as pH-dependent release, time for release in various media (e.g., acid, base, simulated intestinal fluid), release *in vivo,* size, and composition.

In some cases, excipients to encourage spheronization may be used together with the active ingredient to form spheroids. Microcrystalline cellulose and hydrous lactose impalpable are examples of such spheronizing agents. Additionally (or alternatively), the spheroids may contain a water-insoluble polymer, preferably an acrylic polymer; an acrylic copolymer, such as a methacrylic acid-ethyl acrylate copolymer; or ethyl cellulose. In such a formulation, any sustained-release coating present may include a water-insoluble material such as a wax, either alone or in admixture with a fatty alcohol, or shellac or zein.

Spheroids or beads coated with an active ingredient may be prepared, for example, by dissolving the Compound (I) in water, and then spraying the solution onto a substrate, such as a sugar sphere. Optionally, additional ingredients may be added prior to coating the beads in order to assist the active ingredient binding to the substrates, and/or to color the solution, etc. The resulting substrate-active material may be overcoated with a barrier material to separate the Compound (I) from the next coat of material, e.g., a release-retarding material. The barrier material may be a material comprising hydroxypropyl methylcellulose. However, any film-former known in the art may be used. Preferably, the barrier material increases stability during processing and/or shelf-life, without affecting the dissolution rate of the final product.

In order to achieve the desired release characteristics, Compound (I) may be coated with an amount of release-retarding material sufficient to obtain a weight-gain level of about 1 to about 80% (e.g., about 2 to about 40%); however, a greater or lesser amount of release-retarding material may be used depending, for example, on the desired release rate. Moreover, there may be more than one release-retarding material used in the coating, as well as various other pharmaceutical excipients.

The release-retarding material may be in the form of a film coating comprising a dispersion of a hydrophobic polymer. Solvents typically used for application of the release-retarding coating include pharmaceutically acceptable solvents, such as water, alcohols (e.g., methanol or ethanol), methylene chloride, and combinations comprising one or more of the foregoing solvents.

The *in vivo* and/or *in vitro* release profile of the compositions of the invention may be altered; for example, the release profile may be optimised by using more than one release-retarding material, by varying the thickness of the release-retarding material, changing the particular release-retarding material used, altering the relative amounts of release-retarding material, altering the manner in which any plasticizer present is added, varying the amount of plasticizer relative to retardant material, including additional ingredients or excipients, altering the method of manufacture, or by combinations of the foregoing.

In addition to or instead of being present in a matrix, the release-retarding agent can be in the form of a coating. Optionally, a core can be coated, or a gelatin capsule can be further coated, with a sustained and/or delayed and/or prolonged release coating such as those described herein. The coatings may include a sufficient amount of a hydrophobic material to obtain an increase in the weight of the dosage of about 1 to about 80% (e.g., about 2 to about 40%), although the coating can increase the weight of the dosage form by a larger percent depending on the desired release rate, among other variables.

The compositions of the invention preferably release Compound (I) slowly, e.g., when ingested and exposed to gastric fluids, and then to intestinal fluids. The controlled-release profile of the formulations may be altered, for example, by varying the amount of release-retarding agent, e.g., hydrophobic material, by varying the amount of any plasticizer present relative to hydrophobic material, by the inclusion of additional ingredients or excipients, by altering the method of manufacture, or combinations of the foregoing.

The compositions of the invention may be prepared in such a way that, Compound (I) is present in amorphous form or crystalline form. The term "amorphous" is intended to mean consisting of disordered arrangements of molecules which do not possess a distinguishable crystal lattice. A typical process for forming a composition comprising amorphous Compound (I) comprises mixing Compound (I) with water and a pharmaceutically acceptable polymeric carrier, and drying the mixture to form a composition comprising amorphous Compound (I) and the polymeric carrier.

Suitable pharmaceutically acceptable polymeric carriers include, for example, hydroxypropyl cellulose, methyl cellulose, carboxymethyl cellulose, sodium carboxymethyl cellulose, cellulose acetate phthalate, cellulose acetate butyrate, hydroxyethyl cellulose, ethyl cellulose, polyvinyl alcohol, polypropylene, dextran, dextrins, hydroxypropyl-betacyclodextrin, chitosan, lactic/glycolide copolymers, polyorthoester, polyanhydrate, polyvinyl chloride, polyvinyl acetate, ethylene vinyl acetate, lectins, carbopols, silicon elastomers, polyacrylic polymers, maltodextrins, lactose, fructose, inositol, trehalose, maltose, raffinose, polyvinylpyrrolidone (PVP), polyethylene glycol (PEG), and alpha-, beta-, and gamma-cyclodextrins; and combinations of the foregoing carriers.

Preferred polymeric carriers are one or more of polyvinylpyrrolidone, hydroxypropylmethyl cellulose, hydroxypropyl cellulose, methyl cellulose, block copolymers of ethylene oxide and propylene oxide, and polyethylene glycol. The polyvinylpyrrolidone (PVP) typically has an average molecular weight of about 2,500 to about 3,000,000; for example, about 10,000 to about 450,000.

The polymeric carrier is preferably (i) miscible with both Compound (I) free base and its pharmaceutically acceptable salts (especially the hydrochloride salt); (ii) capable of keeping the salt in a homogeneous noncrystalline solid-state dispersion after the water has been removed by evaporation; (iii) chemically inert with respect to Compound (I); and (iv) at least partially water-soluble, and more preferably is fully water-soluble.

Compound (I), the polymeric carrier, and water may be combined in any order. Typically, they are combined in such a manner so as to form a solution of Compound (I) and the polymeric carrier. In forming a solution of the polymeric carrier and water, heating the solution is not generally necessary at lower concentrations; however, it is preferred at higher concentrations, provided that the temperature does not result in decomposition or degradation of any materials. In order to form a clear solution, it is preferable to add Compound (I) after dissolving the polymeric carrier in water, suitably at about 25 to about 100°C; for example, about 45 to about 80°C.

The ratio of Compound (I) to the polymeric carrier can be varied depending, for example, on the precise release profile required. Typical weight ratios of polymeric carrier to Compound (I) are in the range of about 100:1 to about 0.5:1, preferably about 50:1 to about 1:1; such as about 20:1 to about 2:1 (e.g., about 5:1).

Upon formation of the (preferably clear) solution, the process proceeds by recovering the water to form a solid-state dispersion of Compound (I) in the polymeric carrier. Any method of removal of the water that provides a homogeneous solid-state dispersion can be used; suitable methods include evaporation under vacuum, or spray-drying. Methods of evaporation under vacuum include rotary evaporation, static vacuum-drying, and a combination thereof. One skilled in the art of pharmaceutical formulations can readily determine a reasonable temperature at which water can be removed, provided the temperature is not so high as to cause degradation or decomposition of the materials. Typically, evaporation occurs at about 25°C to about 100°C. Evaporation of water should provide a solid state dispersion which is homogeneous and substantially free of water. "Substantially free" is meant to imply that the solid state dispersion typically contains less than 20% by weight of residual water, preferably less than 10%, more preferably less than 5%, most preferably less than 1%.

Any suitable pharmaceutically acceptable excipient can be added to the compositions of the invention. Examples of pharmaceutically acceptable excipients include diluents, vehicles for Compound (I), binders, disintegrants, glidants, sweeteners, compression aids, coloring agents, flavoring agents, suspending agents, dispersing agents, film formers, printing inks, lubricants and/or preservatives. These excipients may be used in a conventional manner, and alone or in any combination.

The pharmaceutical composition may be formulated by conventional methods of admixture such as blending, filling, granulation and compressing. Direct compression and wet granulation are two examples of methods that may be used to formulate the compositions of the invention. These and other methods are described and/or exemplified in more detail hereinafter.

Excipients may be added for numerous reasons; for example, to facilitate manufacture, enhance stability, control release, enhance product characteristics, enhance bioavailability, enhance patient acceptability, and combinations thereof.

Exemplary binders that may be used to help hold the dosage form together include polyvinyl pyrrolidone, hydroxypropyl cellulose, hydroxypropyl methylcellulose, methylcellulose, hydroxyethyl cellulose, sugars, and combinations thereof. Disintegrants (such as croscarmellose sodium) expand when wet, causing tablets to break apart. Lubricants typically aid in the processing of powder materials. Exemplary lubricants include calcium stearate, glycerol behenate, magnesium stearate, mineral oil, polyethylene glycol, sodium stearylfumarate, stearic acid, talc, vegetable oil, zinc stearate, and combinations thereof. An example of a glidant is silicon dioxide.

The formulations described herein may contain a filler such as a water-insoluble or water-soluble filler, or a combination thereof. Typical water-insoluble fillers include silicon dioxide, titanium dioxide, talc, alumina, starch, kaolin, polacrilin potassium, powdered cellulose, microcrystalline cellulose, and combinations thereof. Typical water-soluble fillers include water-soluble sugars and sugar alcohols, preferably lactose, glucose, fructose, sucrose, mannose, dextrose, galactose, the corresponding sugar alcohols and other sugar alcohols, such as mannitol, sorbitol, xylitol, and combinations thereof.

Compound (I) and any optional additives may be prepared as subunits or as pellets; for example, by a melt pelletization technique. In this technique, the Compound (I) in finely divided form is combined with a binder and other optional inert ingredients, and thereafter the mixture is pelletized; e.g., by mechanically working the mixture in a high shear mixer to form the pellets. By the term "pellets", we include pellets, granules, spheres and beads. Thereafter, the pellets can be sieved in order to obtain pellets of the requisite size.

The binder material may also be in particulate form, and typically has a melting point above about 40°C. Suitable binder substances include hydrogenated castor oil, hydrogenated vegetable oil, other hydrogenated fats, fatty alcohols, fatty acid esters, fatty acid glycerides, and combinations thereof.

Oral dosage forms may be prepared to include an effective amount of subunits containing Compound (I), and optionally other active agents in the form of multiparticles or multipellets within a capsule. For example, a plurality of multiparticulates may be placed in a gelatin capsule in an amount sufficient to provide a release profile, as defined above.

Subunits (e.g., in the form of multiparticulates) may be compressed into an oral tablet using conventional tableting equipment and standard techniques. The tablet formulation may include excipients such as, for example, an inert diluent (e.g., lactose); granulating and disintegrating agents (e.g., a cornstarch); binding agents (e.g., starch); and lubricating agents (e.g., magnesium stearate).

Alternatively, subunits containing Compound (I) and optionally containing additional active agents may be subjected to an extrusion process, the resulting extrudate then being shaped into tablets by methods known in the art. The diameter of the extruder aperture or exit port can be adjusted to vary the thickness of the extruded strands. Furthermore, the exit port of the extruder may have any suitable shape; for example, round, oblong or rectangular. The exiting strands can be reduced to particles using any suitable method; for example, with a hot wire cutter or a guillotine.

A melt-extruded multiparticulate system can be, for example, in the form of granules, spheroids, pellets, or the like, depending upon the extruder exit orifice. The terms "melt-extruded multiparticulate(s)", "melt-extruded multiparticulate system(s)" and "melt-extruded particles" are used interchangeably herein, and typically include a plurality of subunits, preferably of similar size and/or shape. The melt-extruded multiparticulates are typically about 0.1 to about 12 mm in length, and about 0.1 to about 5 mm in diameter. In addition, the melt-extruded multiparticulates can be any geometrical shape within this size range. Alternatively, the extrudate can simply be cut into desired lengths, and divided into unit doses of Compound (I), without the need for a pelletization step.

Many of the oral dosage forms described herein contain Compound (I), and optionally additional active agents in the form of particles. Such particles may be compressed into a tablet; may be present in a core element of a coated dosage form, such as a taste-masked dosage form, a press-coated dosage form, or an enteric-coated dosage form; or may be contained in a capsule, osmotic pump dosage form, or other dosage form.

For particles (e.g., powder particles) present in the core element of a coated dosage form, the particles may have a particle size of about 1 µm to about 250 (m, preferably about 25 (m to about 200 (m, more preferably about 35 (m to about 150 (m. The core element typically has a particle size distribution with a median of about 100 (m.

Inconsistencies in size and shape can lead to inconsistent coating. Where the particles containing Compound (I) are of different size and shape, polymeric coating materials such as ethyl cellulose may deposit differently on each particle. Therefore, it is preferable for coated dosage forms that most, if not all, particles of the dosage form have substantially the same size and shape, so that the coating process is better controlled and maintained.

The compositions described herein may be coated with a coating material. The coating typically comprises about 0 to about 90% by weight of the composition. The coating material typically includes a polymer, preferably a film-forming polymer, for example, methyl cellulose, ethyl cellulose, hydroxypropyl cellulose, hydroxypropyl methyl cellulose, hydroxybutyl methyl cellulose, cellulose acetate, cellulose propionate, cellulose acetate propionate, cellulose acetate butyrate, cellulose acetate phthalate, carboxymethyl cellulose, cellulose triacetate, cellulose sulphate sodium salt, poly(methyl methacrylate), poly(ethyl methacrylate), poly(butyl methacrylate), poly(isobutyl methacrylate), poly(hexyl methacrylate), poly(phenyl methacrylate), poly(methyl acrylate), poly(isopropyl acrylate), poly(isobutyl acrylate), poly(octadecyl acrylate), high or low density, polyethylene, polypropylene, poly(ethyleneglycol), poly(ethylene oxide), poly(ethylene terephthalate), poly(vinyl alcohol), poly(vinyl isobutyl ether), poly(vinyl acetate), poly(vinyl chloride), polyvinylpyrrolidone, and combinations thereof.

The coating material may be water-soluble or water-insoluble. For certain applications, such as taste-masking, it is preferable to use a water-insoluble polymer. Suitable water-insoluble polymers include ethyl cellulose or dispersions of ethyl cellulose; acrylic and/or methacrylic ester polymers; cellulose acetates, butyrates or propionates, or copolymers of acrylates or methacrylates having a low quaternary ammonium content; and combinations of the foregoing polymers.

Preferred hydrophobic or water-insoluble polymers for use in the compositions of the invention include, for example, methacrylic acid esters, ethyl cellulose, cellulose acetate, polyvinyl alcohol-maleic anhydride copolymers, (-pinene polymers, glyceryl esters of wood resins, and combinations of the foregoing.

The coating may also include one or more monomeric materials such as sugars (e.g., lactose, sucrose, fructose and mannitol), salts (e.g., sodium chloride and potassium chloride), and organic acids (e.g., fumaric acid, succinic acid, tartaric acid and lactic acid). The coating may also include therein a filler, such as described earlier.

The coating composition may include additives that improve the physical properties of the coating film. For example, the coating composition may comprise a plasticizer. For example, because ethyl cellulose has a relatively high glass transition temperature and does not form flexible films under normal coating conditions, it may be advantageous to add a plasticizer to the ethyl cellulose before using it as a coating material. Generally, the amount of plasticizer included in a coating solution is based on the concentration of the polymer, typically ranging from 0 to about 50% by weight of the coating composition. Suitable concentrations of the plasticizer may be determined by routine experimentation.

Examples of plasticizers for ethyl cellulose and other celluloses include plasticizers such as dibutyl sebacate, diethyl phthalate, triethyl citrate, tributyl citrate, triacetin, acetylated monoglycerides, phthalate esters, castor oil, and combinations thereof.

Examples of plasticizers for acrylic polymers include citric acid esters such as triethyl citrate, tributyl citrate, dibutyl phthalate, 1,2-propylene glycol, polyethylene glycols, propylene glycol, diethyl phthalate, castor oil, triacetin, acetylated monoglycerides, phthalate esters, castor oil, and combinations thereof.

A typical coating comprises (a) a poorly water-permeable component such as an alkyl cellulose (e.g., ethylcellulose), such as AQUACOAT (a 30% solution) or SURELEASE (a 25% solution); and (b) a water-soluble component, e.g., an agent that can form channels through the poorly water-permeable component upon the hydration or dissolution of the soluble component.

Preferably, the water-soluble component (b) is a low-molecular-weight polymeric material; e.g., hydroxyalkylcellulose, hydroxyalkyl(alkylcellulose), carboxymethylcellulose, or salts thereof. Particular examples of these water-soluble polymeric materials include hydroxyethylcellulose, hydroxypropylcellulose, hydroxyethylmethylcellulose, hydroxypropylmethyl cellulose (e.g. METHOCEL), carboxymethylcellulose, sodium carboxymethyl cellulose, and combinations thereof. The water-soluble component (b) is preferably of relatively low molecular weight, preferably less than about 25,000, preferably less than about 21,000.

In the coating, the weight ratio of the water-soluble component (b) to the poorly water-permeable portion (a) is typically about 1:4 to about 2:1, such as about 1:2 to about 1:1; for example, about 2:3. The coating typically constitutes about 1 to about 90% by weight, such as about 2% to about 50%; for example, about 5 to about 30%, of the weight of the total composition.

Preferably, the coating may be a substantially continuous coat, and substantially hole-free. This is particularly advantageous, for example, where the coating provides taste-masking. The phrase "substantially continuous coating" is meant to include a coating that retains a smooth and continuous appearance when magnified 1000 times under a scanning electron microscope, and wherein no holes or breakage of the coating are evident. Typically, the coating is about 0.005 to about 25 µm thick, preferably about 0.05 to about 5 µm.

One or more of the coatings described herein may be used in the compositions of the subject invention. If two or more coatings are present, the coating material used for each coating may be the same, or different.

Any suitable method may be used to apply the coating. Processes that may be used include simple or complex coacervation, interfacial polymerization, liquid drying, thermal and/or ionic gelation, spray-drying, spray-chilling, fluidized bed coating, pan coating and electrostatic deposition. A substantially continuous coating may be achieved, for example, by spray-drying from a suspension or dispersion of Compound (I) in a solution of the coating composition including a polymer in a solvent in a drying gas having a low dew point.

When a solvent is used to apply the coating, the solvent is preferably an organic solvent that is a good solvent for the coating material and a poor solvent for Compound (I). While Compound (I) may partially dissolve in the solvent, it is preferable that the active ingredient precipitate out of the solvent during the spray-drying process more rapidly than the coating material. The solvent may be selected from alcohols such as methanol and ethanol; halogenated hydrocarbons such as dichloromethane (methylene chloride); hydrocarbons such as cyclohexane; and combinations thereof.

The concentration of polymer in the solvent will normally be less than about 75% by weight, typically about 10 to about 30% by weight. After coating, the coated dosage forms are typically allowed to cure for about 1 to about 2 hours, at a temperature of about 50°C to about 60°C.

The dosage form (e.g., a tablet) can be prepared by various conventional mixing, comminution and fabrication techniques readily apparent to those skilled in the chemistry of drug formulations. Examples of such techniques are direct compression (using appropriate punches and dies fitted to a suitable rotary tableting press), injection- or compression-molding using suitable molds fitted to a compression unit, granulation followed by compression, and extrusion into a mold or to an extrudate to be cut into lengths.

When particles or tablets are made by direct compression, the addition of lubricants to the particles/tablets may be helpful and sometimes important to promote powder flow and to prevent capping of the particle (breaking off of a portion of the particle) when the pressure is relieved. Any of the lubricants previously described herein may be used. Preferred lubricants include magnesium stearate and/or sodium stearyl fumarate (typically in a concentration of about 0.1 to about 10%; e.g., about 0.25 to about 3% by weight in the powder mix), and hydrogenated vegetable oil; for example, hydrogenated and refined triglycerides of stearic and palmitic acids may be used at about 1 to about 5% by weight in the powder mix. Additional excipients may be added to enhance powder flowability and reduce adherence. Compositions of the invention made by direct compression are described in more detail in the Examples.

Oral dosage forms may be prepared by including an effective amount of melt-extruded subunits in the form of multiparticles within a capsule. For example, a plurality of the melt-extruded multiparticulates can be placed in a gelatin capsule in an amount sufficient to provide the desired release profile when administered orally. Alternatively, the composition may be in the form of microtablets enclosed inside a gelatin capsule. Microtablets typically have a size of 0.5 to 7 mm in their largest dimension, such as 1 to 6 mm; for example, 3 to 4 mm.

A number of formulations are described below as having preferred components. It is to be understood that any of the components described as being used in one type of formulation may also be used in another type of formulation, even though such components may not be listed as being used in the other formulation. Moreover, the formulations described below may also contain any of the excipients described above, or indeed any of the excipients known in the art.

The compositions of the invention may be in the form of a wax formulation. A wax formulation is a solid dosage form comprising the Compound (I) in a waxy matrix.

The wax material used in the composition of the invention may be, for example, an amorphous wax, an anionic wax, an anionic emulsifying wax, a bleached wax, a carnauba wax, a cetyl ester wax, a beeswax, a castor wax, an emulsifying wax such as a cationic emulsifying wax, a cetrimide emulsifying wax, or a nonionic emulsifying wax, a glycerol behenate, a microcrystalline wax, a nonionic wax, a paraffin, a petroleum wax, a spermaceti wax, a white wax, and combinations of one or more of the foregoing waxes.

A cetyl ester wax suitable for use in the invention typically has a molecular weight of about 470 to about 490, and is a mixture containing primarily esters of saturated fatty alcohols and saturated fatty acids. A wax matrix suitable for use in the compositions of the invention contains carnauba wax and no other waxy material. Another suitable wax matrix includes carnauba wax and glycerol behenates. The wax matrices suitable for use in the invention may be used with or without a coating.

The wax material may be used in the range of about 30 to about 95%, preferably 40 to about 85%, more preferably about 45 to about 80%, most preferably about 50% to about 75% by weight of the total weight of the matrix material. The remainder of the matrix material is typically Compound (I), although other optional components (e.g., fatty acid soaps; see below) may also be present. When a combination of waxes is used, the component waxes can be used in any suitable ratio. For example, if a combination of carnauba wax and glyceryl behenate is used, the relative amounts of each wax is typically about 99 to 60 parts carnauba wax (for example, 99 to about 85 parts) and about 1 to about 40 parts glyceryl behenate (for example, 1 to about 15 parts). In formulations that have a combination of carnauba wax and castor wax, the relative amounts of each wax is typically about 99 to 60 parts carnauba wax (for example, 99 to about 85 parts), and about 1 to about 40 parts castor wax (for example, 1 to about 15 parts). When carnauba wax, glyceryl behenate, and castor wax are present, the carnauba wax typically comprises at least about 85% of the waxy material present, the balance being made up of a combination of glyceryl behenate and castor wax.

Fatty acids and fatty acid soaps may be present in the waxy dosage form. In some cases, the fatty acids and/or fatty acid soaps can replace a portion of the wax material. These optional fatty acids and fatty acid soaps can be those that are generally used in the pharmaceutical industry as tableting lubricants. Such fatty acids and fatty acid soaps include solid fatty acids (for example, fatty acids having about 16 to about 22 carbon atoms), the alkaline earth metal salts thereof (particularly the magnesium and calcium salts), and combinations of the foregoing.

For example, the fatty acid can be stearic acid. The optional fatty acids and fatty soaps, when present, are typically used in amounts of up to about 10% of the total weight of the matrix material, such as about 1 to about 9%; for example, about 2 to about 8%, or about 3 to about 6% of the total weight of the matrix material.

To prepare the wax formulation, the wax or waxes may be melted and used to granulate Compound (I) using melt-granulation techniques. The granulate may be allowed to cool, and then milled to a proper size. Advantageously, the granulate is milled to an average particle size of about 75 µm to about 850 µm, preferably about 150 µm to about 425 µm. The milled granulate may be mixed with optional processing aids. The processing aids include, for example, hydrophobic colloidal silicon dioxide. Hydrophobic silicon dioxide may typically be used in amounts of less than or equal to about 0.5% by weight of the matrix material; however, individual formulations can be varied, as required. The blend of the waxy granulate and the processing aids, if any, may be compressed, and then optionally coated.

The wax formulation may be formulated into any suitable dosage form, for example, coated (e.g., with a functional coating composition or a non-function related coating composition) or uncoated tablets, compressed pellets contained in capsules, or loose-powder or powder-filled capsules.

When the coating composition is a functional coating composition, it typically comprises a water-insoluble component and a water-soluble component. When the coating composition is a non-functional coating composition, it typically comprises a water-soluble component, preferably in the absence of a water-insoluble component. The coating composition may comprise pharmaceutically acceptable dyes, pigments, or mixtures thereof.

As described above, the compositions of the invention may comprise one or more active agents in addition to Compound (I). Therefore, the wax formulation may also include an active agent in addition to Compound (I) in the matrix.

The wax formulations described herein may be made by hot-melting a waxy material to form a melt, and granulating Compound (I) with the melt to form a granulate. The granulate is then typically milled and compressed to form a matrix. The method may further comprise blending the granulate with a processing aid prior to compressing the granulate to form the matrix. The method may further comprise coating the matrix with a functional and/or a non-functional coating.

The compositions of the invention may be in the form of press-coat formulations. Such formulations comprise a core composition containing Compound (I) with a coating composition press-coated on the core. The core composition typically comprises a waxy material containing Compound (I). The coating composition typically comprises a hydrophilic polymer, and optionally Compound (I).

The waxy material of the core composition is typically a hydrophobic waxy material capable of providing the controlled release of Compound (I). Such waxy materials may be, for example, carnauba wax, tribehenin, fatty alcohols (particularly those having 12-24 carbon atoms, such as lauryl alcohol, myristyl alcohol, stearyl alcohol, palmityl alcohol, etc.), fatty acids (particularly those having 12-24 carbon atoms, such as lauric acid, myristic acid, stearic acid, palmitic acid, etc.), polyethylenes, castor wax, C₁₆₋₃₀ fatty acid triglycerides, beeswax, and combinations of one or more of the foregoing waxes.

The hydrophilic polymer of the coating composition is typically chosen so as to aid the controlled release of Compound (I). An example of such a hydrophilic polymer is a film-forming polymer, such as a hydrophilic cellulose polymer, in particular a hydroxyalkyl cellulose polymer. Examples of such hydroxyalkyl cellulose polymers include hydroxyethylcellulose (HEC), hydroxypropylcellulose (HPC), hydroxypropylmethylcellulose (HMPC), hydroxypropylethylcellulose (HPEC), hydroxypropylpropylcellulose (HPPC), hydroxypropylbutylcellulose (HPBC), and combinations of one or more of the foregoing polymers.

Both the core composition and the coating composition may independently include a filler, such as a water-soluble or insoluble filler, or a mixture thereof. Examples of water-insoluble fillers include talc, and calcium salts such as a calcium phosphate, e.g., a dicalcium phosphate. If there is a filler in the coating composition, it can be the same as the filler in the core composition, if any, or different. For example, the core composition may include a water-soluble filler, while the coating composition may include a water-insoluble filler.

Optional excipients can also be present in the core composition and/or the coating composition. Such excipients include lubricants (such as talc and magnesium stearate), glidants (such as fumed or colloidal silica), pH modifiers (such as acids, bases and buffer systems), pharmaceutically useful processing aids, and combinations of one or more of the foregoing excipients. Excipients in the compositions can be the same as those in the core compositions, or different.

In order to form the press-coat formulations, the core composition components (Compound (I), waxy material, and optional excipients) are typically blended together, and compressed into suitable cores. The blending can take place in a suitable order of addition. The cores may be blended by starting with the smallest volume component, and then successively adding the larger volume components. An alternative process is to melt the wax, and blend Compound (I) and optional excipients into the melted wax. Alternatively, Compound (I), wax and any optional excipients can be blended together and then subjected to a temperature at which the wax will melt. Once cooled, the solidified mass can be milled into granules for compaction into cores.

Typically, the core composition is press-coated with the coating composition to form a tablet. The tablet may be further coated with optional additional coatings. The additional coatings can be pH-dependent or pH-independent, aesthetic or functional, and can contain Compound (I) or a different active agent.

If Compound (I) is present in the coating composition, the molar ratio of Compound (I) in the core to Compound (I) in the coating composition is about 500:1 to about 1:10, such as about 100:1 to about 1:5; e.g., about 10:1 to about 1:1.

A preferred press-coat formulation comprises a core composition comprising Compound (I) coated with a coating composition comprising hydroxypropylmethyl cellulose (HPMC). The core composition optionally comprises one or more waxy materials, e.g., carnauba wax; and the coating composition optionally comprises Compound (I). Such press coat formulations may be prepared by press-coating the coating composition onto the core composition.

The compositions of the invention may be formulated using osmotic pump technology. Osmotic pump technology uses osmotic pressure to deliver Compound (I) at a controlled rate. Osmotic pump dosage formulations typically include a semi-permeable membrane surrounding a core that contains at least two components, one component comprising Compound (I), the other comprising an osmotic push layer (an osmotically active expandable driving member), such as an osmotically active polymer. After the dosage form is swallowed, water enters the membrane at a rate primarily determined by the nature of the membrane. This causes the push layer to swell, releasing Compound (I) at a controlled rate through an exit means comprising a passageway or orifice (e.g., a laser-drilled hole) by the action of the osmotically active driving member.

The osmotic pump formulation typically comprises a semipermeable membrane; for example, a capsule, tablet or other dosage form typically having an outer wall comprising a selectively semipermeable material. The selectively permeable material preferably has the following characteristics: (i) it does not adversely affect a host or animal; (ii) it is permeable to the passage of an external aqueous fluid, such as water or biological fluids, while remaining essentially impermeable to the passage of Compound (I); (iii) it is substantially insoluble in body fluids, (iv) it is non-toxic; and (v) it is non-erodible in the environments to which it is subjected.

Representative materials for forming the selectively semipermeable wall include semipermeable homopolymers and copolymers. Suitable materials include, for example, cellulose esters, cellulose monoesters, cellulose diesters, cellulose triesters, cellulose ethers, cellulose ester-ethers, and combinations thereof. These cellulosic polymers have a degree of substitution (DS) on their anhydroglucose unit of greater than 0 to about 3. The "degree of substitution" is the average number of hydroxyl groups originally present on the anhydroglucose unit that have been replaced by a substituting group, or converted into another group. The anhydroglucose unit can be partially or completely substituted with semipermeable polymer-forming groups such as acyl, alkanoyl, aroyl, alkenyl, alkoxy, halogen, carboalkyl, alkylcarbamate, alkylcarbonate, alkylsulfonate and alkylsulfamate.

Other selectively semipermeable materials include, for example, cellulose acylate; cellulose diacylate; cellulose triacylate; cellulose acetate; cellulose diacetate; cellulose triacetate; mono-, di- and tri-cellulose alkanylates; mono-, di- and tri- alkenylates; mono-, di- and tri-aroylates; and combinations of the foregoing materials. Exemplary polymers include cellulose acetate having a DS of 1.8 to 2.3, and an acetyl content of about 32 to about 40%; cellulose diacetate having a DS of 1 to 2, and an acetyl content of about 21 to about 35%; and cellulose triacetate having a DS of 2 to 3, and an acetyl content of about 34 to about 45%. Other examples of cellulosic polymers include cellulose propionate having a DS of 1.8 and a propionyl content of about 38.5%; cellulose acetate propionate having an acetyl content of about 1.5 to about 7%, and a propionyl content of about 39 to about 42%; and cellulose acetate propionate having an acetyl content of about 2.5% to about 3%, an average propionyl content of about 39 to about 45% and a hydroxyl content of about 2.8% to about 5.4%. Further exemplary cellulosic polymers include cellulose acetate butyrate having a DS of 1.8, an acetyl content of about 13 to about 15% and a butyryl content of about 34% to about 39%; and cellulose acetate butyrate having an acetyl content of about 2 to about 29.5%, a butyryl content of about 17 to about 53%, and a hydroxyl content of about 0.5% to about 4.7%. Still further examples of suitable cellulosic polymers include cellulose triacylates have a DS of 2.9 to 3, such as cellulose trivalerate, cellulose trilaurate, cellulose tripalmitate, cellulose trioctanoate, and cellulose tripropionate; cellulose diesters having a DS of 2.2 to 2.6, such as cellulose disuccinate, cellulose dipalmitate, cellulose dioctanoate, and cellulose dicaprylate; mixed cellulose esters, such as cellulose acetate valerate, cellulose acetate succinate, cellulose propionate succinate, cellulose acetate octanoate, cellulose valerate palmitate, and cellulose acetate heptanoate; and combinations of the foregoing cellulosic polymers.

Other potentially suitable semipermeable polymers include, for example, acetaldehyde dimethyl cellulose acetate, cellulose acetate ethylcarbamate, cellulose acetate methylcarbamate, cellulose dimethylaminoacetate, semipermeable polyamides, semipermeable polyurethanes, semipermeable polysulfanes, semipermeable sulfonated polystyrenes, cross-linked selectively semipermeable polymers formed by the coprecipitation of a polyanion and a polycation, semipermeable silicon rubbers, semipermeable polystyrene derivatives, semipermeable poly(sodium styrenesulfonate), semipermeable poly(vinylbenzyltrimethyl)ammonium chloride polymers; and combinations comprising the foregoing polymers, including combinations with one or more of the selectively permeable materials listed in the preceding paragraph.

The osmotically expandable driving member (or osmotic push layer) of the osmotic pump dosage form is typically a swellable and expandable inner layer. The materials suitable for forming the osmotic push layer include polymeric materials and/or polymeric materials blended with osmotic agents, both of which typically interact with water or a biological fluid, absorb the fluid, and swell or expand to an equilibrium state in the presence of the fluid without dissolving. Preferably, the polymer should exhibit the ability to retain a significant fraction of absorbed fluid in the polymer molecular structure. Such polymers may be gel polymers that can swell or expand to a very high degree; for example, exhibiting an about 2 to about 50-fold volume increase.

Suitable swellable, hydrophilic polymers, also known as osmopolymers, can be non-cross-linked or lightly cross-linked. The cross-links can be covalent or ionic bonds with the polymer. The polymer may be of plant, animal or synthetic origin. Polymeric materials useful for the present purpose include poly(hydroxyalkyl methacrylate) having a molecular weight of about 5,000 to about 5,000,000; polyvinylpyrrolidone having a molecular weight of about 10,000 to about 360,000; anionic and cationic hydrogels; poly(electrolyte) complexes; poly(vinyl alcohol) having a low acetate residual; a swellable mixture of agar and carboxymethyl cellulose; a swellable composition comprising methyl cellulose mixed with a sparingly crosslinked agar; a water-swellable copolymer produced by a dispersion of finely divided copolymer of maleic anhydride with styrene, ethylene, propylene, or isobutylene; water-swellable polymers of N-vinyl lactams; and combinations of the foregoing polymers.

Other gelable, fluid-absorbing and retaining polymers useful for forming the osmotic push layer include pectins having a molecular weight ranging about 30,000 to about 300,000; polysaccharides such as agar, acacia, karaya, tragacanth, algins and guar; poly(carboxylic acids) and their salt derivatives; polyacrylamides; water-swellable indene maleic anhydride polymers; polyacrylic acid having a molecular weight of about 80,000 to about 200,000; polyethylene oxide polymers having a molecular weight of about 100,000 to about 5,000,000 (but may be higher); starch graft copolymers, polyanion and polycation exchange polymers, starch-polyacrylonitrile copolymers, acrylate polymers with water absorbability of about 100 to about 600 times their original weight; diesters of polyglucan; a mixture of cross-linked polyvinyl alcohol and poly(N-vinyl-2-pyrrolidone); zein (available as prolamine); poly(ethylene glycol) having a molecular weight of about 4,000 to about 100,000; and combinations of the foregoing polymers.

The osmotically expandable driving layer of the osmotic pump dosage form may further contain an osmotically effective compound (osmagent) that can be used as is, or blended homogeneously or heterogeneously with the swellable polymer discussed above. Such osmagents are typically osmotically effective solutes that are soluble in the fluid absorbed into the swellable polymer, and exhibit an osmotic pressure gradient across the semipermeable wall against an exterior fluid.

Suitable osmagents include, for example, solid compounds such as magnesium sulfate, magnesium chloride, sodium chloride, lithium chloride, potassium sulfate, sodium sulfate, mannitol, urea, sorbitol, inositol, sucrose, glucose, and combinations thereof. The osmotic pressure of the osmagents is typically about 0 to about 500 atm, but may be higher.

The swellable, expandable polymer of the osmotically expandable driving layer may, in addition to providing a driving source for delivering Compound (I) from the dosage form, also function as a supporting matrix for an osmotically effective compound (or osmagent). The osmotic compound may be homogeneously or heterogeneously blended with the polymer to yield the desired expandable wall or expandable pocket. A typical osmotic pump dosage form may comprise about 20 to about 90% by weight of polymer and about 80 to about 10% by weight of osmotic compound, preferably about 35 to about 75% by weight of polymer and about 65 to about 25% by weight of osmotic compound, based on the total weight of the formulation.

The Compound (I) in the osmotic pump dosage form may be formulated in any suitable manner; for example, as a thermo-responsive formulation in which Compound (I) is dispersed in a thermo-responsive composition. Alternatively, the osmotic pump dosage form may contain a thermo-responsive element comprising a thermo-responsive composition at the interface of the osmotic push layer and Compound (I) composition. Representative thermo-responsive compositions (including their melting points in parentheses) are cocoa butter (32°C-34°C); cocoa butter and 2% beeswax (35°C-37°C); propylene glycol monostearate and distearate (32°C-35°C); hydrogenated oils, such as hydrogenated vegetable oil (36°C-37.5°C); 80% hydrogenated vegetable oil and 20% sorbitan monopalmitate (39°C-39.5°C); 80% hydrogenated vegetable oil and 20% polysorbate 60 (36°C-37°C); 77.5% hydrogenated vegetable oil, 20% sorbitan trioleate, 2.5% beeswax and 5.0% distilled water (37°C-38°C); mono-, di-, and triglycerides of acids having 8-22 carbon atoms, including saturated and unsaturated acids such as palmitic, stearic, oleic, linoleic and archidonic; triglycerides of saturated fatty acids with mono- and diglycerdies (34°C-35.5°C); propylene glycol mono- and distearates (33°C-34°C); partially hydrogenated cottonseed oil (35°C-39°C); block copolymers of polyoxyalkylene and propylene glycol; block copolymers of 1,2-butylene oxide and ethylene oxide; block copolymers of propylene oxide and ethylene oxide, hardened fatty alcohols and fats (33°C-36°C); hexadienol and hydrous lanolin triethanolamine glyceryl monostearate (38°C); eutectic mixtures of mono-, di-, and triglycerides (35°C-39°C); WITEPSOL H15, triglyceride of saturated vegetable fatty acid with monoglycerides (33.5°C-35.5°C); WITEPSOL H32, free of hydroxyl groups (31°C-33°C); WITEPSOL W25, having a saponification value of 225-240 (33.5°C-35.5°C); WITEPSOL E75, having a saponification value of 220-230 (37°C-39°C); a polyalkylene glycol such as polyethylene glycol 1000; a linear polymer of ethylene oxide (38°C-41°C); polyethylene glycol 1500 (38°C-41°C); polyethylene glycol monostearate (39°C-42.5°C); 33% polyethylene glycol 1500, 47% polyethylene glycol 6000 and 20% distilled water (39°C-41°C); 30% polyethylene glycol 1500, 40% polyethylene glycol 4000 and 30% polyethylene glycol 400 (33°C-38°C); mixtures of mono-, di- and triglycerides of saturated fatty acids having 11 to 17 carbon atoms (33°C-35°C); and mixtures of the foregoing.

The thermo-responsive compositions, including thermo-responsive carriers, are thought to be useful for storing Compound (I) in a solid composition at a temperature of about 20°C to about 33°C, maintaining an immiscible boundary at the swelling composition interface, and dispensing the agent in a flowable composition at a temperature greater than about 33°C, preferably about 33°C to about 40°C.

When the Compound (I)-containing thermo-responsive formulations described above is used, the integrity of the semi-permeable membrane, which is also present in such osmotic pump formulations, is preferably not compromised (e.g., melted or eroded) by the presence of the thermo-responsive formulations.

Compound (I) in the osmotic pump dosage form may be formulated by any suitable techniques known in the art; for example, by wet granulation or fluid bed granulation, as described in more detail below.

Firstly, Compound (I) and the ingredients comprising the Compound (I) layer are blended using an organic solvent, such as isopropyl alcohol-ethylene dichloride 80:20 v/v (volume: volume) as the granulation fluid. Other granulating fluids, such as 100% denatured alcohol, may be used for this purpose. The ingredients forming the Compound (I) layer are individually passed through a screen such as a 40-mesh screen, and then thoroughly blended in a mixer. Next, other ingredients comprising the Compound (I) layer are dissolved in a portion of the granulation fluid. Then, the latter-prepared wet blend is slowly added to the Compound (I) blend with continual mixing in the mixer. The granulating fluid is added until a wet blend is produced, whose wet mass is then forced through a screen such as a 20-mesh screen, and then onto oven trays. The blend is dried for about 18 to about 24 hours at about 30°C to about 50°C. The dry granules are then sized with a screen such as a 20-mesh screen. Next, a lubricant is passed through a screen such as an 80-mesh screen, and added to the dry granule blend. The mixture is put into milling jars, and mixed in a jar mill for about 1 to about 15 minutes. The push layer may also be made by the same wet granulation techniques. The compositions are pressed into their individual layers in a KILIAN press-layer press.

Another manufacturing process that can be used for providing the Compound (I) layer and the osmotically expandable driving layer comprises blending the powdered ingredients for each layer independently in a fluid bed granulator. After the powdered ingredients are dry-blended in the granulator, a granulating fluid (e.g., poly(vinylpyrrolidone) in water, denatured alcohol, 95:5 ethyl alcohol/water, or blends of ethanol and water) is sprayed onto the powders. Optionally, the ingredients can be dissolved or suspended in the granulating fluid. Typically, the coated powders are then dried in a granulator. This process granulates the ingredients present therein, while adding the granulating fluid. After the granules are dried, a lubricant such as stearic acid or magnesium stearate is added to the granulator. The granules for each separate layer may then be pressed in the manner described above for the wet granulation method.

The osmotic push Compound (I) formulation and osmotic push layer of the osmotic push dosage form may also be manufactured by mixing Compound (I) with composition-forming ingredients, and pressing the composition into a solid lamina. In a further alternative method of manufacture, Compound (I), any other composition-forming ingredients and a solvent are typically mixed into a solid, or a semisolid, by methods such as ball-milling, calendaring, stirring or roll-milling; and then pressed into a preselected layer-forming shape. Next, a layer of composition comprising an osmopolymer and an optional osmagent is typically placed in contact with the layer comprising Compound (I). The layering of the first layer comprising Compound (I), and the second layer comprising the osmopolymer and optional osmagent composition may be accomplished by using a conventional layer press technique.

The semipermeable wall can be applied by molding, spraying or dipping the shapes of the pressed bilayer into wall-forming materials. An air suspension coating procedure that includes suspending and tumbling the two layers in a current of air until the wall-forming composition surrounds the layers may also be used to form the semi-permeable wall of the osmotic formulations.

The dispenser of the osmotic pump dosage form may be, for example, in the form of a hard or soft capsule. The capsule may also be osmotic.

The hard capsule may be composed of two parts, a cap and a body, which are typically fitted together after the body (which is generally larger than the cap) is filled with Compound (I). The hard capsule may be fitted together by slipping or telescoping the cap section over the body section, thus completely surrounding and encapsulating Compound (I).

The soft capsule of the osmotic pump dosage form may be a one-piece soft capsule. Typically, the soft capsule comprises a sealed construction encapsulating Compound (I). The capsule may be made by various processes, such as the plate process, the rotary die process, the reciprocating die process, and the continuous process.

Materials useful for forming the capsule of the osmotic pump dosage form may be commercially available materials including gelatin (typically having a viscosity of about 5 to about 30 millipoises and a bloom strength up to about 150 grams; or gelatin having a bloom value of about 150 to about 250), a composition comprising gelatin, glycerine, water and titanium dioxide; a composition comprising gelatin, erythrosine, iron oxide and titanium dioxide; a composition comprising gelatin, glycerine, sorbitol, potassium sorbate and titanium dioxide; a composition comprising gelatin, acacia, glycerine, and water; and combinations thereof. Commercially available gelatin capsules (e.g., CAPSUGEL) may also be used.

The semipermeable wall-forming composition may be applied to the Compound (I)-containing component and/or to the exterior surface of the capsule in laminar arrangement by molding, forming, air spraying, dipping or brushing. Alternative techniques that can be used for applying the semipermeable wall include air suspension procedures and pan-coating procedures. For example, an air suspension procedure includes suspending and tumbling the capsule arrangement in a current of air and a semipermeable wall-forming composition until the wall surrounds and coats the capsule. The procedure can be repeated with a different semipermeable wall-forming composition to form a semipermeable laminated wall.

Exemplary solvents suitable for manufacturing the semipermeable wall include inert inorganic and organic solvents that do not adversely harm the materials used in the osmotic pump formulations, e.g., the capsule wall, Compound (I), the thermo-responsive composition, the expandable member, or the final dispenser. Such solvents include aqueous solvents, alcohols, ketones, esters, ethers, aliphatics hydrocarbons, halogenated solvents, cycloaliphatics, aromatics, heterocyclic solvents, and combinations thereof. Particular solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride, nitroethane, nitropropane, tetrachloroethane, ethyl ether, isopropyl ether, cyclohexane, cyclooctane, benzene, toluene, naphtha, 1,4-dioxane, tetrahydrofuran, water, and mixtures thereof such as acetone and water, acetone and methanol, acetone and ethyl alcohol, methylene dichloride and methanol, and ethylene dichloride, methanol, and combinations of the foregoing.

The exit means or hole in the osmotic pump formulations for releasing Compound (I) may be produced during manufacture, or in use. For example, the exit means or hole can be formed by mechanical or laser drilling, or by eroding an erodible element in the wall, such as a gelatin plug. The orifice can be a polymer inserted into the semipermeable wall, whose polymer is a (micro)porous polymer that typically has at least one (micro)pore.

An example of a formulation for the controlled release of Compound (I) in the stomach and gastrointestinal tract is one in which Compound (I) is dispersed in a polymeric matrix that is water-swellable, rather than merely hydrophilic. Such water-swellable matrices typically also have an erosion rate that is substantially slower than their swelling rate, and release Compound (I) primarily by diffusion.

The rate of diffusion of Compound (I) from the matrix can be modified by varying numerous characteristics of the formulation. For example, the rate of diffusion of Compound (I) can be slowed by increasing the Compound (I) particle size, by the choice of polymer used in the matrix, and/or by the choice of molecular weight of the polymer. The matrix is typically a relatively high molecular weight polymer that swells upon ingestion, preferably to a size that is at least about twice its unswelled volume; and that might, in addition, promote gastric retention. Upon swelling, the matrix may convert over a prolonged period of time (such as about 1 to about 48 hours; e.g., about 2 to about 24 hours, or about 3 to about 12 hours) from a glassy or crystalline polymer to a polymer that is rubbery in consistency.

Typically, penetrating fluid causes release of Compound (I) in a gradual and prolonged manner by the process of solution diffusion, i.e., dissolution of Compound (I) in the penetrating fluid, and diffusion of the dissolved drug back out of the matrix.

Typically, the matrix itself is solid prior to administration; and, once administered, remains undissolved in (i.e., is not eroded by) the gastric fluid for a period of time sufficient to permit the majority of Compound (I) to be released in a controlled manner (as defined by the release profiles described above) by solution diffusion. Therefore, the rate-limiting factor in the release of Compound (I) is believed to be controlled diffusion of Compound (I) from the matrix rather than erosion, dissolving or chemical decomposition of the matrix.

The water-swellable polymer that forms the matrix is a polymer that is nontoxic, swells in a dimensionally unrestricted manner upon absorption of water (and/or other fluids), and provides for sustained release of incorporated Compound (I). Examples of suitable polymers include, for example, cellulose polymers and their derivatives (such as hydroxyethylcellulose, hydroxypropylcellulose, carboxymethylcellulose, and microcrystalline cellulose); polysaccharides and their derivatives; polyalkylene oxides, polyethylene glycols, chitosan, poly(vinyl alcohol), polysaccharide gums, maleic anhydride copolymers, poly(vinyl pyrrolidone), starch and starch-based polymers, poly(2-ethyl-2-oxazoline), poly(ethyleneimine), polyurethane hydrogels, crosslinked polyacrylic acids and their derivatives; copolymers of the foregoing polymers, including block copolymers and grafted polymers (e.g., PLURONIC and TECTONIC, which are polyethylene oxide-polypropylene oxide block copolymers); and mixtures thereof.

As used herein, unless otherwise stated, the terms "cellulose" and "cellulosic" denote a linear polymer of anhydroglucose. Suitable cellulosic polymers include, for example, alkyl-substituted cellulosic polymers that ultimately dissolve in the gastrointestinal (GI) tract in a predictably delayed manner. Specific examples are methylcellulose, hydroxymethylcellulose, hydroxyethylcellulose, hydroxypropylcellulose, hydroxypropylmethylcellulose, and carboxymethylcellulose. The viscosity of suitable alkyl-substituted cellulosic polymers is typically about 100 to about 110,000 centipoise as a 2% aqueous solution at 20°C, or about 1,000 to about 4,000 centipoise as a 1% aqueous solution at 20°C. Exemplary alkyl-substituted celluloses are hydroxyethylcellulose and hydroxypropylmethylcellulose. A specific example of a hydroxyethylcellulose is NATRASOL 250HX NF.

Suitable polyalkylene oxides are those having the properties described above for alkyl-substituted cellulose polymers. An example of a polyalkylene oxide is poly(ethylene oxide) (PEO), a term used herein to denote a linear polymer of unsubstituted ethylene oxide. Suitable PEO polymers typically have molecular weights of greater than about 4,000,000, preferably about 4,500,000 to about 10,000,000, more preferably about 5,000,000 to about 8,000,000. Preferred polyethylene oxides are those with a weight average molecular weight in the range of about 1 x 10⁵ to about 1 x 10⁷, preferably about 9 x 10⁵ to about 8 x 10⁶. Suitable PEOs typically have a viscosity of about 50 to about 2,000,000 centipoise as a 2% aqueous solution at 20°C. Two specific examples of PEOs are POLYOX NF, grade WSR Coagulant, molecular weight 5 million; and grade WSR 303, molecular weight 7 million.

Examples of suitable polysaccharide gums are natural and modified (semi-synthetic) polysaccharide gums such as dextran, xanthan gum, gellan gum, welan gum and rhamsan gum.

Suitable crosslinked polyacrylic acids include those whose properties are the same as or similar to those described above for alkyl-substituted cellulose and polyalkylene oxide polymers. Typically, such crosslinked polyacrylic acids have a viscosity of about 4,000 to about 40,000 centipoise as a 1% aqueous solution at 25°C. Three specific examples are CARBOPOL NF grades 971P, 974P and 934P. Further examples include polymers known as WATER LOCK polymers, which are starch/acrylates/acrylamide copolymers.

As mentioned above, the hydrophilicity and water-swellability of the polymers discussed above cause Compound (I)-containing matrices to swell in size in the gastric cavity due to ingress of water and/or other fluids. This swelling promotes retention of the matrices in the stomach during the fed phase. The hydrophilicity and water-swellability also cause the matrices to become slippery, which provides resistance to peristalsis and further promotes their retention in the stomach.

The release rate of Compound (I) from the matrix is primarily dependent upon the rate of water absorption and the rate at which Compound (I) dissolves and diffuses from the swollen polymer, which in turn is related to the solubility and dissolution rate of Compound (I), Compound (I) particle size and Compound (I) concentration in the matrix. Additionally, because these matrix-forming polymers typically dissolve very slowly in gastric fluid, the matrix maintains its physical integrity over at least a substantial period of time, typically for at least 70 or 80% of the dosing period, and in many cases at least 90% and even over 100% of the dosing period. Generally, the particles then slowly dissolve or decompose. Complete dissolution or decomposition may not occur until 24 hours or more after administration; although in many cases, complete dissolution or decomposition will occur within 10 to 24 hours after the dosing period.

The swellable matrix dosage forms may include additives that impart a small degree of hydrophobic character, to further retard the release rate of Compound (I) into the gastric fluid. Examples of such release rate retardants are glyceryl monostearate, fatty acids, and salts of fatty acids (e.g., sodium myristate). Typically, the weight ratio of additive to Compound (I) is in the range of about 1:10 to about 10:1; for example, about 1:5 to about 5:1.

The amount of polymer relative to Compound (I) may vary, depending on the precise nature of the desired release profile, its molecular weight, and excipients that may be present in the formulation. However, the amount of polymer will be sufficient so that the polymeric matrix will remain substantially intact until all of Compound (I) is released. The term "substantially intact" is used herein to denote a polymeric matrix in which the polymer portion substantially retains its size and shape without deterioration due to becoming solubilized in the gastric fluid, or due to breakage into fragments or small particles.

The water-swellable polymers can be used individually, or in combination. Certain combinations will often provide a more controlled release of Compound (I) than their components when used individually. Such combinations include cellulose-based polymers (e.g., hydroxyethyl cellulose or hydroxypropyl cellulose) or poly(ethylene oxide) combined with gums, (e.g., xanthan gum).

The benefits of the swellable matrix dosage form are typically achieved over a wide range of Compound (I) loadings; for example, weight ratios of Compound (I) to polymer of about 0.001:1 to about 10:1. Typical loadings (expressed in terms of the weight percent of Compound (I) relative to Compound (I) and polymer combined) are about 0.001% to about 50%, preferably about 0.01% to about 40%, such as about 0.1% to about 30%; for example, about 1% to about 20%.

The swellable matrix formulations also find significant utility when administered to a subject who is in the digestive state (also referred to as the postprandial or "fed" mode). The postprandial mode is distinguishable from the interdigestive (or "fasting") mode by distinct patterns of gastroduodenal motor activity, which determine the gastric retention or gastric transit time of the stomach contents.

Thus, administration of the formulation during the digestive state results in localization of Compound (I) release in the stomach and small intestine, and reduces and/or prevents substantial colonic degradation, inactivation, or loss of bioavailability.

Juvenile and elderly patients often require dosage forms that are easy to swallow; for example, to reduce the risk of choking upon administration, and/or to improve patient compliance. The compositions of the invention may be in the form of easily administrable dosage forms, making them more suitable for patient compliance. Such easily administrable formulations include, for example, sprinkle dosage forms, taste-masked liquid dosage forms, fast-dissolve dosage forms, and chewable dosage forms:

It is to be understood that any of the easily administrable dosage forms described below may comprise any of the formulations described above in order to provide a composition that has the desired release profile of Compound (I) according to the subject invention.

An example of a chewable dosage form is a Compound (I)-containing chewable tablet. Such a chewable tablet comprises a chewable base and, optionally, a sweetener. The chewable base typically comprises an excipient such as mannitol, sorbitol, lactose, or a combination thereof. The optional sweetener used in the chewable dosage form may be, for example, sucrose, liquid glucose, sorbitol, dextrose, isomalt, liquid maltitol, aspartame, lactose, or a combination thereof. In certain cases, the chewable base and the sweetener may be the same component. The chewable base and optional sweetener typically comprise about 50% to about 90% by weight of the total weight of the chewable dosage form.

The chewable dosage form may additionally contain preservatives, agents that retard and/or prevent adhesion to the oral cavity and crystallization of sugars, flavoring agents, souring agents, coloring agents, and combinations of one or more of the foregoing. Glycerin, lecithin, hydrogenated palm oil or glyceryl monostearate may be used as a protecting agent of crystallization of the sugars, typically in an amount of about 0.01 to about 2% by weight of the total weight of the ingredients. Such protecting agents help to prevent adhesion to the oral cavity, and improve the soft property or chewability of the dosage form. Additionally or alternatively, isomalt or liquid maltitol may be used to enhance the chewing properties of the chewable dosage form.

The method for making the chewable dosage form comprising Compound (I) described above is similar to the method used to make soft confectionary. Such a method typically involves the formation of a boiled sugar-corn syrup blend to which is added a frappe mixture. The boiled sugar-corn syrup blend may be prepared from sugar and corn syrup blended in parts by weight ratio of 90:10 to 10:90. This blend may be heated to temperatures above 120°C to remove water, and form a molten mass. The frappe mixture may be prepared from gelatin, egg albumen, milk proteins such as casein, vegetable proteins such as soy protein, and the like, which are added to a gelatin solution and rapidly mixed at ambient temperature to form an aerated, sponge-like mass. The frappe mixture is then added to the molten candy base and mixed until homogenous, typically at temperatures between 60°C to about 120°C. A matrix, tablet or other formulation containing Compound (I) may then be added to the mix at a temperature of about 60°C to about 90°C, whereupon additional ingredients such as flavors, colorants, and preservatives may be added. The formulation is then typically cooled, and formed to pieces of desired dimensions.

Fast-dissolving dosage forms may comprise microparticles and one or more effervescent agents, enabling the dosage forms to rapidly disintegrate in the mouth while providing adequate taste-masking. Alternatively, rapidly dissolving dosage forms may contain an active agent and a matrix that includes a non-direct compression filler and a lubricant. US Patent No. 5,178,878 and US Patent No. 6,221,392 provide teachings regarding fast-dissolve dosage forms.

Typical fast-dissolve dosage forms for use in the subject invention include a mixture incorporating a water- and/or saliva-activated effervescent agent, a disintegration agent, and microparticles. The microparticles typically incorporate Compound (I) together with a protective material substantially encompassing the Compound (I). The term "substantially encompassing" includes the meaning that the protective material substantially shields Compound (I) from contact with the environment outside the microparticle. Thus, each microparticle may incorporate a discrete mass of Compound (I) covered by a coating of the protective material, in which case the microparticle can be referred to as a "microcapsule" or a "microtablet". Alternatively or additionally, each microparticle may have Compound (I) dispersed or dissolved in a matrix of the protective material, optionally coated by a coating composition as described herein.

The mixture including the microparticles and an effervescent agent is typically present as a tablet of a size and shape adapted for direct oral administration to a patient. The tablet is substantially completely disintegrable upon exposure to water and/or saliva. The effervescent disintegration agent is present in an amount effective to aid disintegration of the tablet, and to provide a distinct sensation of effervescence when the tablet is placed in the mouth of a patient.

The effervescent sensation is typically not only pleasant to the patient, but also tends to stimulate saliva production, thereby providing additional water to aid in further effervescent action. Thus, once the tablet is placed in the patient's mouth, it will generally disintegrate rapidly and substantially completely without any voluntary action by the patient. Thus, even if the patient does not chew the tablet, disintegration should proceed rapidly. Upon disintegration of the tablet, the microparticles are released, and can be swallowed as a slurry or suspension of the microparticles. The microparticles are thus transferred to the patient's stomach for dissolution in the digestive tract and systemic distribution of the Compound (I).

The terms "effervescent agent" and "disintegration agent" include compounds that evolve gas. Such agents may evolve gas by means of chemical reactions that take place upon their exposure to water and/or to saliva in the mouth. The bubble- or gas-generating reaction is most often the result of the reaction of a soluble acid source and an (alkali metal) carbonate source. The reaction of these two general classes of compounds produces carbon dioxide gas upon contact with the water in saliva.

Such saliva-/water-activated materials should be kept in a generally anhydrous state with little or no absorbed moisture, or in a stable, hydrated form, since exposure to water will prematurely disintegrate the tablet. For example, the dosage form may be stored in substantially air-tight packaging prior to administration.

The acid source may be any acid source that is safe for human consumption, and may generally include food acids, acid anhydrides, and acid salts. Food acids include citric acid, tartaric acid, malic acid, fumaric acid, adipic acid, succinic acids, etc. Because these acids are directly ingested, their overall solubility in water is less important than if the formulations were intended to be dissolved in a glass of water. Acid anhydrides and acid salts of the above-described acids may also be used. Acid salts may include sodium, dihydrogen phosphate, disodium dihydrogen pyrophosphate, acid citrate salts, and sodium acid sulfite.

The carbonate source includes dry solid carbonate, and bicarbonate salts such as sodium bicarbonate, sodium carbonate, potassium bicarbonate and potassium carbonate, magnesium carbonate and sodium sesquicarbonate, sodium glycine carbonate, L-lysine carbonate, arginine carbonate, amorphous calcium carbonate, and combinations thereof.

While the effervescent disintegration agent is typically one that, upon a reaction, forms carbon dioxide, this is not essential. Effervescent disintegration agents that evolve oxygen or other gasses that are safe for human patients may also be used.

When the effervescent agent includes two mutually reactive components, such as an acid source and a carbonate source, it is preferable that both components react substantially completely. Therefore, an equimolar ratio of acid and carbonate sources is preferable. For example, if the acid used is diprotic, then either twice the molar amount of a mono-reactive carbonate base, or an equal molar amount of a di-reactive base, should be used for complete neutralization to be realized. However, the amount of either the acid or carbonate source may exceed the amount of the other component. This may be useful to enhance the taste and/or performance of a tablet containing an excess of either component. In such cases, it is acceptable that the additional amount of either component remain unreacted.

The fast-dissolving dosage forms (e.g., tablets) typically contain an amount of effervescent disintegration agent effective to aid in the rapid and complete disintegration of the tablet when orally administered. By "rapid", it is understood that the tablets should disintegrate in the mouth of a patient in less than 10 minutes, such as about 15 seconds to about 7 minutes; for example, about 30 seconds to about 5 minutes. Disintegration time in the mouth can be measured by observing the disintegration time of the tablet in water at about 37°C. The tablet is immersed in the water without forcible agitation. The disintegration time is the time from immersion to substantially complete dispersion of the tablet, as determined by visual observation. As used herein, the term "complete disintegration" of the tablet does not require dissolution or disintegration of the microcapsules, or other discrete inclusions.

In order to achieve such disintegration, the amount of effervescent agent or disintegration agent typically used in the fast-dissolve dosage forms is about 5% to about 50% by weight of the final composition, preferably about 15% to about 40% by weight, more preferably about 20% to about 30% by weight.

The tablets described above can be manufactured by well-known tableting procedures.

As mentioned above, each microparticle typically incorporates Compound (I) in conjunction with a protective material. The microparticle may be provided as a microcapsule, microtablet, or matrix-type microparticle. Microcapsules may incorporate a discrete mass of Compound (I) surrounded by a discrete, separately observable coating of the protective material. Conversely, in a matrix-type particle, Compound (I) is dissolved, suspended or otherwise dispersed throughout the protective material. Certain microparticles may include attributes of both microcapsules and matrix-type particles. For example, a microparticle may incorporate a core incorporating a dispersion of Compound (I) in a first protective material and a coating of a second protective material, which may be the same or different from the first protective material surrounding the core. Alternatively, a microparticle may incorporate a core consisting essentially of Compound (I) and a coating incorporating the protective material, the coating itself having some Compound (I) dispersed therein. The microparticles typically have a mean diameter of about 75 to about 600 µm, preferably about 150 to about 500 µm; for example, about 200 to about 450 µm. The microparticles may be about 200 to about 30 mesh (US standard size); for example, about 100 to about 35 mesh.

The protective materials suitable for use in the fast-dissolve dosage forms described above typically include polymers that are conventionally utilized in the formation of microparticles such as matrix-type microparticles, microtablets and microcapsules. Among these are cellulosic materials such as naturally occurring cellulose, synthetic cellulose derivatives, acrylic polymers, and vinyl polymers. Other simple polymers, such as proteinaceous materials (e.g., gelatin, polypeptides), and natural and synthetic shellacs and waxes, may also be used. Protective polymers may also include ethylcellulose, methylcellulose, carboxymethyl cellulose and acrylic resin material.

When a coating is used in the above fast-dissolve dosage forms, it typically comprises at least about 5% by weight based on the total weight of the resulting particles, preferably at least about 10% by weight. The upper limit of the protective coating material used is generally less critical. In certain embodiments, it is possible to use a coating that is greater than 100 percent of the weight of the core, providing a relatively thick coating. However, the amount of coating material should not be so great that it impedes the release of a therapeutically effective amount of Compound (I) before defecation of the dosage form.

An example of a fast-dissolve dosage form is a hard, compressed, rapidly dissolvable dosage form adapted for direct oral dosing. Such a dosage form typically includes Compound (I), often in the form of a protected particle, and a matrix. The matrix typically includes a filler and a lubricant; however, it may include other additional ingredients. Although the dosage form is adapted to rapidly dissolve in the mouth of a patient, it has a friability of about 2% or less when tested according to the USP. Generally, the dosage form will also have a hardness of at least about 1.5 or 2.0 kP. Not only does the dosage form dissolve quickly, it does so in a way that provides a positive organoleptic sensation to the patient. In particular, the dosage form dissolves with a minimum of unpleasant grit, which is tactilely very inconsistent with organoleptic sensation of the dosage form.

The filler typically comprises a non-direct compression filler. Exemplary fillers include, for example, non-direct compression sugars, and sugar alcohols. Such sugars and sugar alcohols include dextrose, mannitol, sorbitol, lactose, and sucrose. Dextrose, for example, can exist as either a direct compression sugar, i.e., a sugar that has been modified to increase its compressibility, or a non-direct compression sugar. The percentage of filler is typically in the range of about 25 to about 98% by weight of the microparticles, preferably about 50 to about 95%; for example, about 60 to about 90%.

In the fast-dissolve dosage forms discussed above, a relatively high proportion of lubricant is typically used. Lubricants, in particular, hydrophobic lubricants such as magnesium stearate, may be used in an amount of about 0.25 to about 5% by weight of the formulation, preferably about 1 to about 3% by weight; for example, about 1.5 to about 2% by weight. Despite the use of this relatively high percentage weight of lubricant, the formulations typically exhibit excellent compressibility, hardness, and rapid dissolution within the mouth.

Hydrophobic lubricants include, for example, alkaline earth metal stearates, stearic acid, mineral and vegetable oils, glyceryl behenate, sodium stearyl fumarate, and combinations thereof. Hydrophilic lubricants may also be used.

The hard, compressed fast-dissolve dosage forms typically have a hardness of at least about 1.5 kP, and are designed to dissolve spontaneously and rapidly in the mouth of a patient in less than about 90 seconds to thereby liberate the particles. Preferably, the dosage form will dissolve in less than about 60 seconds, and even more preferably in about 30 to about 45 seconds. This measure of hardness is based on the use of small tablets of less than about 0.25 inches in diameter. A hardness of at least about 2.0 kP is preferred for larger tablets. Direct compression techniques are preferred for the formation of these tablets.

Sprinkle dosage forms are another form of easily administered formulations that may be used in the compositions of the invention. Sprinkle dosage forms typically comprise Compound (I) in the form of pellets, granules, microtablets or microcapsules, optionally having functional or non-functional coatings. In use, the patient or caregiver can sprinkle the particulate/pelletized dose into drink, or onto soft food. A sprinkle dosage form may comprise particles having a mean diameter of about 10 to about 100 µm in their major dimension; for example, about 50 to 70 µm.

An example of a sprinkle dosage form is an easily openable capsule enclosing a plurality of Compound (I)-containing micropellets. Each of the micropellets typically comprises a seed coated with a first coating mixture of Compound (I) and polyvinylpyrrolidone; and a second coating mixture of about 90 to about 70% by weight of the mixture of a non-hydrophilic polymer (e.g., ethyl cellulose), and about 10 to about 30% by weight of the mixture of a hydrophilic polymer (e.g., hydroxypropyl methyl cellulose). For example, the second coating mixture may comprise about 3 parts ethylcellulose to about 1 part hydroxypropylcellulose. The weight of the second coating mixture is about 5-10% of the weight of the micropellets before the second coating is applied. Optionally, the second coating contains Compound (I).

The polyvinylpyrrolidone used in the first coating typically has a molecular weight of about 30,000 to about 50,000, e.g., about 40,000. The seed of the sprinkle dosage form may be a sugar seed, and have a mesh size of 60/80.

Taste-masked dosage forms are another form of easily administered formulations that may be used in the compositions of the invention. The taste-masked dosage form may be liquid or solid.

A solid taste-masked dosage form typically comprises a core element comprising Compound (I) and a coating material surrounding the core element. The core element comprising Compound (I) is typically in the form of a (micro)particle, (micro)tablet, (micro)capsule, amorphous solid, pellet, granule, powder, or matrix. The core element may include carriers or excipients, fillers, flavoring agents, stabilizing agents and/or colorants in addition to Compound (I).

The taste-masked dosage form typically includes about 50 to about 99% by weight, preferably about 65 to about 95% by weight, for example about 80 to about 90% by weight of the Compound (I)-containing core element, based on the total weight of the dosage form. The taste-masked dosage form typically includes about 1 to about 50% by weight, preferably about 5 to about 35% by weight; for example, about 10 to about 20% by weight of the coating material surrounding the core element, based on the total weight of the dosage form.

The core element typically includes about 20 to about 90% by weight of a supplementary component selected from waxes, water-insoluble polymers, enteric polymers, and partially water-soluble polymers; other suitable pharmaceutical excipients; and combinations thereof.

The core element optionally includes carriers or excipients, fillers, flavoring agents, stabilizing agents, colorants, and combinations thereof. Suitable fillers include, for example, insoluble materials such as silicon dioxide, titanium dioxide, talc, alumina, starch, kaolin, polacrilin potassium, powdered cellulose, and microcrystalline cellulose; and combinations comprising one or more of the foregoing fillers. Soluble fillers include, for example, mannitol, sucrose, lactose, dextrose, sodium chloride, sorbitol, and combinations comprising one or more of the foregoing fillers. The filler may be present in amounts of up to about 75% by weight, based on the total weight of the dosage form.

The core element may be in the form of a powder, for example, having a particle size range of about 35 µm to about 125 µm. Such small particle size facilitates a substantially non-gritty feel in the mouth. Small particle size also minimizes break-up of the particles in the mouth, e.g., by the teeth. When in form of a powder, the taste-masked dosage form may be administered directly into the mouth; or mixed with a carrier such as water, or semi-liquid compositions such as syrups, yogurt, and the like. However, the taste-masked Compound (I) may be provided in any suitable unit dosage form.

The coating material of the taste-masked formulation may take a form that provides a substantially continuous coating, and that provides taste-masking. The coating may also provide the controlled release of Compound (I). The polymer used in the taste-masked dosage form coating may be a water-insoluble polymer such as, for example, ethyl cellulose. The coating material of the taste masked dosage form may further include a plasticizer.

A method of preparing taste-masked pharmaceutical formulations such as powdered formulations typically includes mixing a core element and a coating material in a diluent, and spray-drying the mixture to form a taste-masked formulation. Spray-drying of the pharmaceutically active ingredient and polymer in the solvent typically involves spraying a stream of air into an atomized suspension, optionally in a drying chamber; causing the solvent to evaporate, and leaving Compound (I) coated with the polymer coating material.

For a solvent such as methylene chloride, the solvent concentration in the drying chamber is typically maintained at about 40,000 to about 100,000 parts per million of organic solvent. The spray-drying process for such solvents may be conducted at a process temperature of about 5°C to about 35°C. Spray-drying of the dosage forms may be undertaken utilizing either rotary, pneumatic or pressure atomizers located in either a co-current or mixed-flow spray dryer; or variations thereof. The drying gas may be heated or cooled to control the rate of drying. A temperature below the boiling point of the solvent may be used. Inlet temperatures may be about 40 to about 120°C, and outlet temperatures about 5°C and 35°C.

The coat formation may be optimized to meet the needs of the material or application. Controlling the process parameters such as temperature, solvent concentration, spray dryer capacity, atomizing air pressure, droplet size, viscosity, and total air pressure in the system and the solvent system allows for the formation of a range of coats, ranging from dense, continuous, non-porous coats to more porous microcapsule/polymer matrices.

A post-treatment step may be used to remove any residual solvent. The post treatment may include a post-drying step including drying the final product on a tray and/or at a bed temperature sufficient to remove excess solvent, but not degrade the Compound (I). The drying temperature is preferably in the range of about 35°C to about 40°C. Once completed, the product may be collected by a suitable method, such as collection by sock filters, or cyclone collection.

An exemplary chewable taste-masked dosage form comprises a microcapsule of about 10 µm to about 1.5 mm in diameter having a core comprising Compound (I), and a polymer mixture coating having sufficient elasticity to withstand chewing. The polymeric mixture coating typically comprises about 30 to about 70% by weight of a polymer that forms a polymeric film at temperatures of at least about 30°C (e.g., ethylcellulose), and about 30 to about 70% by weight of a copolymer that forms a polymeric film at temperatures less than about 25°C. The polymeric mixture coating is adapted so that the dosage form exhibits the release profiles discussed earlier in this specification.

The copolymer that forms a polymeric film at temperatures less than about 25°C is typically a methacrylic acid ester copolymer (having, for example, a weight average molecular weight of about 800,000) or a styrene acrylate copolymer.

The core of the taste-masked Compound (I) dosage form described above may comprise a diluent and/or a plasticizer. Suitable plasticizers include, but are not limited to, polyethylene glycol, triacetin, vinylpyrrolidone, diethyl phthalate, dibutyl sebacate, a citric acid ester, and combinations thereof.

Solid taste-masked dosage forms (e.g., polymer-coated Compound (I) powder) may be reconstituted as suspensions in a liquid vehicle such as water before usage. This is advantageous in that the reconstitutable solid taste-masked dosage forms typically have a longer shelf life than many liquid taste-masked dosage forms; and the suspensions, once reconstituted, have adequate taste-masking.

The subject invention provides the use of an orally deliverable pharmaceutical composition as defined in the claims for the treatment of a neurological and/or a psychiatric condition.

By the term "a neurological and/or a psychiatric condition", we include all conditions deriving from a pathology of the nervous system. Particular examples of such conditions are described in more detail below.

The phrase "the treatment of a neurological and/or a psychiatric condition" is intended to include use for the acute, chronic and/or prophylactic treatment of neurological, neuropsychiatric, psychiatric and neurodegenerative disease.

Accordingly, there are numerous conditions that may be treated by administering or using the compositions of the invention.

The present invention is useful for disorders selected from schizophrenia; refractory, intractable or chronic schizophrenia; emotional disturbance; psychotic disorder; mood disorder; bipolar disorder; mania; depression; endogenous depression; major depression; melancholy and refractory depression; dysthymic disorder; cyclothymic disorder; anxiety disorder; somatoform disorder; factitious disorder; dissociative disorder; sexual disorder; eating disorder; sleep disorder; adjustment disorder; substance-related disorder; anhedonia; delirium; cognitive impairment; cognitive impairment associated with neurodegenerative diseases; cognitive impairment caused by neurodegenerative diseases; cognitive impairment of schizophrenia; cognitive impairment caused by refractory, intractable or chronic schizophrenia; vomiting; motion sickness; obesity; migraine; pain (ache); mental retardation; autism disorder; Tourette's disorder; tic disorder; attention-deficit/hyperactivity disorder; conduct disorder; and Down's syndrome.

The compositions of the invention may comprise one or more active agents in addition to Compound (I).

For example, the compositions of the invention may comprise another atypical antipsychotic agent (e.g., aripiprazole, olanzapine, quetiapine, risperidone, amisulpride, clozapine, chlorpromazine, or haloperidol decanoate), antiparkinsonian agents (e.g., L-DOPA, Dopamine Agonists), sedatives (e.g., a benzodiazepine sedative or non-barbiturate sedative), anxiolytics (e.g., benzodiazepines such as lorazepam, chlordiazepoxide, oxazepam, clorazepate, diazepam, and alprazolam), antidepressants, and mood stabilizers (e.g., lamotrigine, lithium, valproate, carbamazepine, and oxcarbazepine).

The antiparkinsonian agents may be used to treat the tardive dyskinesia associated with neuroleptic use. Also called "side-effect medication", antiparkinsonian agents are indicated when muscle side-effects of the atypical antipsychotics make patients uncomfortable. Antiparkinsonian agents are typically anticholinergic drugs, examples including benztropine mesylate, trihexyphenidyl, procyclidine, and amantadine.

Suitable antidepressants include tricyclic antidepressants (such as amitriptyline, imipramine, doxepin, and clomipramine), monoamine oxidase A or B inhibitors (such as phenelzine and tranylcypromine), tetracyclic antidepressants (e.g., maprotiline), and serotonin reuptake inhibitors such as fluoxetine, cipramil, S-cipramil, paroxetine, and sertraline hydrochloride, serotonin and noradrenaline reuptake inhibitors such as venlafaxine and duloxetine, noradrenaline reuptake inhibitors such as reboxetine and viloxazine, and all other classes of antidepressants.

Of course, the Compound (I) formulations described herein may be used for the treatment of numerous other conditions in addition to schizophrenia. Such conditions may require treatment by different additional active agents (in addition to Compound (I)) than those described above in relation to the treatment of schizophrenia.

The invention will now be illustrated by the following non-limiting Examples.

### Example 1: Compound (I) Compositions

30 mg direct compression (DC) and wet granulation (WG) controlled-release tablets are manufactured as described below.

### Direct Compression Tablets

The ingredients set out in Table 3 below are blended together in a planetary mixer for 5 minutes. The blend is compressed in a rotary tabletting machine, using 7.0 mm diameter round n/c punches. The tablet breaking strength is 2.5 kp to 3.5 kp.

**Table 3: Direct Compression Composition**

| Ingredient | % | tablet mg | batch g |
|---|---|---|---|
| Compound (I) | 20 | 30 | 100 |
| Methocel (registered trademark) K4M | 35 | 52.5 | 175 |
| Avicel (registered trademark) PH 200 | 44 | 66 | 220 |
| Sodium Stearyl Fumarate | 1 | 1.5 | 5 |
| | 100 | 150 | 500 |

### Wet Granulation Tablets

The ingredients set out in Table 4 below, except for sodium stearyl fumarate, are blended together in a planetary mixer for 5 minutes prior to wet granulation with purified water. The moist powders are dried in a fluid bed drier at an inlet temperature of 70°C for 15 minutes. The dried granule has a loss on drying value of 2.5% w/w. The granules are sieved through an 850 µm screen and blended for 1 minute with the sodium stearyl fumarate. The blend is compressed at 150 mg in a rotary tabletting machine, using 7.0 mm diameter round n/c punches. The tablet breaking strength is 5.0 kp to 6.0 kp.

**Table 4: Wet Granulation Composition**

| Ingredient | % | tablet mg | batch g |
|---|---|---|---|
| Compound (I) | 20 | 30 | 100 |
| Methocel (registered trademark) K4M | 35 | 52.5 | 175 |
| Avicel (registered trademark) PH 200 | 39 | 58.5 | 195 |
| PVPK30 | 5 | 7.5 | 25 |
| Sodium Stearyl Fumarate | 1 | 1.5 | 5 |
| | 100 | 150 | 500 |

| | | | |
|---|---|---|---|
| PVPK30: polyvinylpyrrolidone K30 | | | |

The contents disclosed in any publication cited herein, including patents and patent applications, are hereby incorporated in their entireties by reference, to the extent that they have been disclosed herein.

This application is based on US provisional patent application Nos. 61/471,911 and 61/580,540, the contents of which are incorporated by reference in full herein.

### Embodiments

Embodiments of the invention include the following items 1 to 30:
[Item 1] A medicament comprising
   (I) a compound which is 7-[4-(4-benzo[b]thiophen-4-yl-piperazin-1-yl)butoxy]-1H-quinolin-2-one or a salt thereof, and
   (II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, in combination.
[Item 2] The medicament of Item 1, which is a composition comprising
   (I) a compound which is 7-[4-(4-benzo[b]thiophen-4-yl-piperazin-1-yl)butoxy]-1H-quinolin-2-one or a salt thereof, and
   (II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug.
[Item 3] The medicament of Item 1, which comprises
   (I) a composition comprising a compound which is 7-[4-(4-benzo[b]thiophen-4-yl-piperazin-1-yl)butoxy]-1H-quinolin-2-one or a salt thereof, and
   (II) a composition comprising at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug and, a tricyclic antidepressant, a tetracyclic antidepressant, an antipsychotic drug an anti-ADHD drug,
   wherein the composition of (I) is used in combination with the composition of (II).
[Item 4] The medicament of any one of Items 1 to 3, wherein said drug of (II) is a mood stabilizer.
[Item 5] The medicament of Item 4, wherein said mood stabilizer is at least one drug selected from lithium, sodium valproate, divalproex sodium, carbamazepine, oxcarbazepine, zonisamide, lamotrigine, topiramate, gabapentin, levetiracetam, clonazepam, phenytoin, pregabalin, thyroid hormone, tiagabine, omega-3 fatty acid and salts thereof.
[Item 6] The medicament of any one of Items 1 to 3, wherein said drug of (II) is a serotonin reuptake inhibitor.
[Item 7] The medicament of Item 6, wherein said serotonin reuptake inhibitor is at least one drug selected from the group consisting of fluoxetine, citalopram, fluvoxamine, paroxetine, sertraline, escitalopram and salts thereof.
[Item 8] The medicament of any one of Items 1 to 3, wherein said drug of (II) is a norepinephrine reuptake inhibitor.
[Item 9] The medicament of Item 8, wherein said norepinephrine reuptake inhibitor is at least one drug selected from the group consisting of reboxetine, atomoxetine, bupropion and salts thereof.
[Item 10] The medicament of any one of Items 1 to 3, wherein said drug of (II) is a serotonin and norepinephrine reuptake inhibitor.
[Item 11] The medicament of Item 10, wherein said serotonin and norepinephrine reuptake inhibitor is at least one drug selected from the group consisting of venlafaxine, duloxetine, milnacipran, desvenlafaxine and salts thereof.
[Item 12] The medicament of any one of Items 1 to 3, wherein said drug of (II) is a noradrenergic and specific serotonergic antidepressant.
[Item 13] The medicament of Item 12, wherein said noradrenergic and specific serotonergic antidepressant is at least one drug selected from the group consisting of mirtazapine and a salt thereof.
[Item 14] The medicament of any one of Items 1 to 3, wherein said drug of (II) is an antianxiety drug.
[Item 15] The medicament of Item 14, wherein said antianxiety drug is at least one drug selected from the group consisting of a benzodiazepine antianxiety drug and a serotonin 5-HT1A receptor agonist antianxiety drug.
[Item 16] The medicament of Item 15, wherein said benzodiazepine antianxiety drug is at least one drug selected from the group consisting of diazepam, lorazepam, chlordiazepoxide, cloxazolam, clotiazepam, alprazolam, etizolam, oxazolam and salts thereof, and said serotonin 5-HT1A receptor agonist antianxiety drug is at least one drug selected from the group consisting of tandospirone, buspirone and salts thereof.
[Item 17] The medicament of any one of Items 1 to 3, wherein said drug of (II) is an anti-ADHD drug.
[Item 18] The medicament of Item 17, wherein said anti-ADHD drug is at least one drug selected from the group consisting of methylphenidate, dexmethylphenidate, atomoxetine, dextroamphetamine, mixed amphetamine salts, modafinil, guanfacine, clonidine and salts thereof.
[Item 19] The medicament of any one of Items 1 to 18 for use in the prophylaxis or treatment of a central nervous system disease.
[Item 20] The medicament of Item 19, wherein the central nervous system disease is a central nervous system disease selected from the group consisting of schizophrenia, treatment-resistant, refractory or chronic schizophrenia, emotional disturbance, psychotic disorder, mood disorder, bipolar disorder (e.g., bipolar disorder type I and bipolar disorder type II), mania, depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, acute stress disorder), somatoform disorder (e.g., hysteria, somatization disorder, conversion disorder, pain disorder, hypochondriasis), factitious disorder, dissociative disorder, sexual disorder (e.g., sexual dysfunction, sexual desire disorder, sexual arousal disorder, erectile dysfunction), eating disorder (e.g., anorexia nervosa, bulimia nervosa), sleep disorder, adjustment disorder, substance-related disorder (e.g., alcohol abuse, alcohol intoxication and drug addiction, stimulant intoxication, narcotism), anhedonia (e.g., iatrogenic anhedonia, anhedonia of a psychic or mental cause, anhedonia associated with depression, anhedonia associated with schizophrenia), delirium, cognitive impairment, cognitive impairment associated with Alzheimer's disease, Parkinson's disease, and other neurodegenerative diseases, cognitive impairment caused by Alzheimer's disease, Parkinson's disease and associated neurodegenerative diseases, cognitive impairment in schizophrenia, cognitive impairment caused by treatment-resistant, refractory or chronic schizophrenia, vomiting, motion sickness, obesity, migraine, pain, mental retardation, autistic disorder (autism), Tourette's disorder, tic disorder, attention deficit hyperactivity disorder, conduct disorder and Down's syndrome.
[Item 21] A pharmaceutical composition comprising the medicament of any one of Items 1 to 18 and at least one pharmacologically acceptable carrier.
[Item 22] A method for producing a pharmaceutical composition, comprising mixing the medicament of any one of Items 1 to 18 with a pharmacologically acceptable carrier.
[Item 23] A kit comprising
   (I) a medicament containing a compound which is 7-[4-(4-benzo[b]thiophen-4-yl-piperazin-1-yl)butoxy]-1H-quinolin-2-one or a salt thereof, and
   (II) a medicament containing at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug.
[Item 24] The kit of Item 23, which is a kit for use in the prophylaxis or treatment of a central nervous system disease.
[Item 25] A method for preventing or treating a disease in a mammal, comprising
   administering an effective amount of a compound which is 7-[4-(4-benzo[b]thiophen-4-yl-piperazin-1-yl)butoxy]-1H-quinolin-2-one or a salt thereof to the mammal, and
   administering an effective amount of (II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, to the mammal.
[Item 26] The method of Item 25, wherein said disease is a central nervous system disease.
[Item 27] Use of (I) a compound which is 7-[4-(4-benzo[b]thiophen-4-yl-piperazin-1-yl)butoxy]-1H-quinolin-2-one or a salt thereof and (II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, for the production of a medicament for the treatment of a central nervous system disease by a combination treatment using said drug of (II) together with said compound of (I), wherein said compound of (I) and said drug of (II) are formulated as a part of a single drug, or formulated as individual drugs to be administered simultaneously or at different time points.
[Item 28] Use of (II) at least one drug selected from the group consisting of a mood stabilizer, a serotonin reuptake inhibitor, a norepinephrine reuptake inhibitor, a serotonin and norepinephrine reuptake inhibitor, a noradrenergic and specific serotonergic antidepressant, an antianxiety drug, a tricyclic antidepressant, a tetracyclic antidepressant, an antipsychotic drug and an anti-ADHD drug, together with a compound which is 7-[4-(4-benzo[b]thiophen-4-yl-piperazin-1-yl)butoxy]-1H-quinolin-2-one or a salt thereof, for the treatment of a central nervous system disease.
[Item 29] A medicament for use in the prophylaxis or treatment of a central nervous system disease comprising a compound which is 7-[4-(4-benzo[b]thiophen-4-yl-piperazin-1-yl)butoxy]-1H-quinolin-2-one or a salt thereof, and at least one drug selected from the group consisting of lithium, sodium valproate, divalproex sodium, carbamazepine, oxcarbazepine, zonisamide, lamotrigine, topiramate, gabapentin, levetiracetam, clonazepam, phenytoin, pregabalin, thyroid hormone, tiagabine, omega-3 fatty acid, fluoxetine, citalopram, fluvoxamine, paroxetine, sertraline, escitalopram, reboxetine, atomoxetine, bupropion, venlafaxine, duloxetine, milnacipran, desvenlafaxine, mirtazapine, diazepam, lorazepam, chlordiazepoxide, cloxazolam, clotiazepam, alprazolam, etizolam, oxazolam, tandospirone, buspirone, methylphenidate, dexmethylphenidate, dextroamphetamine, mixed amphetamine salts, modafinil, guanfacine, clonidine and salts thereof, in combination.
[Item 30] The medicament of Item 29, wherein the central nervous system disease is a central nervous system disease selected from the group consisting of schizophrenia, treatment-resistant, refractory or chronic schizophrenia, emotional disturbance, psychotic disorder, mood disorder, bipolar disorder (e.g., bipolar disorder type I and bipolar disorder type II), mania, depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, acute stress disorder), somatoform disorder (e.g., hysteria, somatization disorder, conversion disorder, pain disorder, hypochondriasis), factitious disorder, dissociative disorder, sexual disorder (e.g., sexual dysfunction, sexual desire disorder, sexual arousal disorder, erectile dysfunction), eating disorder (e.g., anorexia nervosa, bulimia nervosa), sleep disorder, adjustment disorder, substance-related disorder (e.g., alcohol abuse, alcohol intoxication and drug addiction, stimulant intoxication, narcotism), anhedonia (e.g., iatrogenic anhedonia, anhedonia of a psychic or mental cause, anhedonia associated with depression, anhedonia associated with schizophrenia), delirium, cognitive impairment, cognitive impairment associated with Alzheimer's disease, Parkinson's disease, and other neurodegenerative diseases, cognitive impairment caused by Alzheimer's disease, Parkinson's disease and associated neurodegenerative diseases, cognitive impairment in schizophrenia, cognitive impairment caused by treatment-resistant, refractory or chronic schizophrenia, vomiting, motion sickness, obesity, migraine, pain, mental retardation, autistic disorder (autism), Tourette's disorder, tic disorder, attention deficit hyperactivity disorder, conduct disorder and Down's syndrome.

## Claims

1. A medicament comprising
(I) a compound which is 7-[4-(4-benzo[b]thiophen-4-yl-piperazin-1-yl)butoxy]-1H-quinolin-2-one or a salt thereof, and
(II) one or more antianxiety drugs, in combination,
wherein the medicament comprises said compound (I) and said drug (II) in a single preparation, or
the medicament comprises a composition comprising said compound (I) and a composition comprising said drug (II), which are separately formulated, and the composition of said compound (I) is used in combination with the composition of said drug (II).

2. The medicament of claim 1, which is a composition comprising
(I) a compound which is 7-[4-(4-benzo[b]thiophen-4-yl-piperazin-1-yl)butoxyl-1H-quinolin-2-one or a salt thereof, and
(II) one or more antianxiety drugs.

3. The medicament of claim 1, which comprises
(I) a composition comprising a compound which is 7-[4-(4-benzo[b]thiophen-4-yl-piperazin-1-yl)butoxy]-1H-quinolin-2-one or a salt thereof, and
(II) a composition comprising one or more antianxiety drugs, wherein the composition of (I) is used in combination with the composition of (II).

4. The medicament of any one of claims 1 to 3, wherein said antianxiety drug is at least one drug selected from the group consisting of a benzodiazepine antianxiety drug and a serotonin 5-HT1A receptor agonist antianxiety drug,
said benzodiazepine antianxiety drug preferably being at least one drug selected from the group consisting of diazepam, lorazepam, chlordiazepoxide, cloxazolam, clotiazepam, alprazolam, etizolam, oxazolam and salts thereof,
and said serotonin 5-HT1A receptor agonist antianxiety drug preferably being at least one drug selected from the group consisting of tandospirone, buspirone and salts thereof.

5. The medicament of any one of claims 1 to 4 for use in the prophylaxis or treatment of a central nervous system disease.

6. The medicament of claim 5, wherein the central nervous system disease is a central nervous system disease selected from the group consisting of schizophrenia, treatment-resistant, refractory or chronic schizophrenia, emotional disturbance, psychotic disorder, mood disorder, bipolar disorder (e.g., bipolar disorder type I and bipolar disorder type II), mania, depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, acute stress disorder), somatoform disorder (e.g., hysteria, somatization disorder, conversion disorder, pain disorder, hypochondriasis), factitious disorder, dissociative disorder, sexual disorder (e.g., sexual dysfunction, sexual desire disorder, sexual arousal disorder, erectile dysfunction), eating disorder (e.g., anorexia nervosa, bulimia nervosa), sleep disorder, adjustment disorder, substance-related disorder (e.g., alcohol abuse, alcohol intoxication and drug addiction, stimulant intoxication, narcotism), anhedonia (e.g., iatrogenic anhedonia, anhedonia of a psychic or mental cause, anhedonia associated with depression, anhedonia associated with schizophrenia), delirium, cognitive impairment, cognitive impairment associated with Alzheimer's disease, Parkinson's disease, and other neurodegenerative diseases, cognitive impairment caused by Alzheimer's disease, Parkinson's disease and associated neurodegenerative diseases, cognitive impairment in schizophrenia, cognitive impairment caused by treatment-resistant, refractory or chronic schizophrenia, vomiting, motion sickness, obesity, migraine, pain, mental retardation, autistic disorder (autism), Tourette's disorder, tic disorder, attention deficit hyperactivity disorder, conduct disorder and Down's syndrome.

7. A pharmaceutical composition comprising the medicament of any one of claims 1 to 4 and at least one pharmacologically acceptable carrier.

8. A kit comprising
(I) a medicament containing a compound which is 7-[4-(4-benzo[b]thiophen-4-yl-piperazin-1-yl)butoxy]-1H-quinolin-2-one or a salt thereof, and
(II) one or more antianxiety drugs.

9. The kit of claim 8, which is a kit for use in the prophylaxis or treatment of a central nervous system disease.

10. A medicament for use in the prophylaxis or treatment of a central nervous system disease comprising (I) a compound which is 7-[4-(4-benzo[b]thiophen-4-yl-piperazin-1-yl)butoxy]-1H-quinolin-2-one or a salt thereof, and (II) at least one drug selected from the group consisting of diazepam, lorazepam, chlordiazepoxide, cloxazolam, clotiazepam, alprazolam, etizolam, oxazolam, tandospirone, buspirone, in combination, wherein the medicament comprises said compound (I) and said drug (II) in a sigle preparation, or
the medicament comprises a composition comprising said compound (I) and a composition comprising said drug (II), which are separately formulated, and the composition of said compound (I) is used in combination with the composition of said drug (II).

11. The medicament of claim 10, wherein the central nervous system disease is a central nervous system disease selected from the group consisting of schizophrenia, treatment-resistant, refractory or chronic schizophrenia, emotional disturbance, psychotic disorder, mood disorder, bipolar disorder (e.g., bipolar disorder type I and bipolar disorder type II), mania, depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, acute stress disorder), somatoform disorder (e.g., hysteria, somatization disorder, conversion disorder, pain disorder, hypochondriasis), factitious disorder, dissociative disorder, sexual disorder (e.g., sexual dysfunction, sexual desire disorder, sexual arousal disorder, erectile dysfunction), eating disorder (e.g., anorexia nervosa, bulimia nervosa), sleep disorder, adjustment disorder, substance-related disorder (e.g., alcohol abuse, alcohol intoxication and drug addiction, stimulant intoxication, narcotism), anhedonia (e.g., iatrogenic anhedonia, anhedonia of a psychic or mental cause, anhedonia associated with depression, anhedonia associated with schizophrenia), delirium, cognitive impairment, cognitive impairment associated with Alzheimer's disease, Parkinson's disease, and other neurodegenerative diseases, cognitive impairment caused by Alzheimer's disease, Parkinson's disease and associated neurodegenerative diseases, cognitive impairment in schizophrenia, cognitive impairment caused by treatment-resistant, refractory or chronic schizophrenia, vomiting, motion sickness, obesity, migraine, pain, mental retardation, autistic disorder (autism), Tourette's disorder, tic disorder, attention deficit hyperactivity disorder, conduct disorder and Down's syndrome.

12. A medicament comprising (I) a compound which is 7-[4-(4-benzo[b]thiophen-4-yl-piperazin-1-yl)butoxy]-1H-quinolin-2-one or a salt thereof, for use in the treatment of a central nervous system disease in a patient being administered with (II) one or more antianxiety drugs which preferably is at least one drug selected from the group consisting of a benzodiazepine antianxiety drug and a serotonin 5-HT1A receptor agonist antianxiety drug,
said benzodiazepine antianxiety drug preferably being at least one drug selected from the group consisting of diazepam, lorazepam, chlordiazepoxide, cloxazolam, clotiazepam, alprazolam, etizolam, oxazolam and salts thereof,
and said serotonin 5-HT1A receptor agonist antianxiety drug preferably being at least one drug selected from the group consisting of tandospirone, buspirone and salts thereof.

13. A medicament comprising (II) one or more antianxiety drug which preferably is at least one drug selected from the group consisting of a benzodiazepine antianxiety drug and a serotonin 5-HT1A receptor agonist antianxiety drug,
said benzodiazepine antianxiety drug preferably being at least one drug selected from the group consisting of diazepam, lorazepam, chlordiazepoxide, cloxazolam, clotiazepam, alprazolam, etizolam, oxazolam and salts thereof,
and said serotonin 5-HT1A receptor agonist antianxiety drug preferably being at least one drug selected from the group consisting of tandospirone, buspirone and salts thereof, for use in the treatment of a central nervous system disease in a patient being administered with (I) a compound which is 7-[4-(4-benzo[b]thiophen-4-yl-piperazin-1-yl)butoxy]-1H-quinolin-2-one or a salt thereof.

14. The medicament for use according to either claim 12 or 13, wherein the treatment is the treatment of a central nervous system diseasee selected from the group consisting of schizophrenia, treatment-resistant, refractory or chronic schizophrenia, emotional disturbance, psychotic disorder, mood disorder, bipolar disorder (e.g., bipolar disorder type I and bipolar disorder type II), mania, depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, acute stress disorder), somatoform disorder (e.g., hysteria, somatization disorder, conversion disorder, pain disorder, hypochondriasis), factitious disorder, dissociative disorder, sexual disorder (e.g., sexual dysfunction, sexual desire disorder, sexual arousal disorder, erectile dysfunction), eating disorder (e.g., anorexia nervosa, bulimia nervosa), sleep disorder, adjustment disorder, substance-related disorder (e.g., alcohol abuse, alcohol intoxication and drug addiction, stimulant intoxication, narcotism), anhedonia (e.g., iatrogenic anhedonia, anhedonia of a psychic or mental cause, anhedonia associated with depression, anhedonia associated with schizophrenia), delirium, cognitive impairment, cognitive impairment associated with Alzheimer's disease, Parkinson's disease, and other neurodegenerative diseases, cognitive impairment caused by Alzheimer's disease, Parkinson's disease and associated neurodegenerative diseases, cognitive impairment in schizophrenia, cognitive impairment caused by treatment-resistant, refractory or chronic schizophrenia, vomiting, motion sickness, obesity, migraine, pain, mental retardation, autistic disorder (autism), Tourette's disorder, tic disorder, attention deficit hyperactivity disorder, conduct disorder and Down's syndrome.
